# EUROPEAN PATENT SPECIFICATION

(11) **EP 4 329 674 B1**
(45) Date of publication and mention of the grant of the patent: **16.07.2025**
(21) Application number: 22722564.6
(22) Date of filing: 25.04.2022
(51) Int. Cl.: A61F 2/90, A61F 2/24

(54) **TRANSCATHETER DEVICES FOR TREATMENT OF A HEART**
TRANSKATHETERVORRICHTUNGEN ZUR BEHANDLUNG EINES HERZENS
DISPOSITIFS TRANSCATHÉTERS DE TRAITEMENT D'UN COEUR

(30) Priority: 29.04.2021 US 202163181565 P
(43) Date of publication of application: 06.03.2024
(73) Proprietor: Edwards Lifesciences Innovation (Israel) Ltd., 3079892 Caesarea (IL)
(72) Inventor: HOFFER, Eran, 563007 Yahud (IL); BRAUON, Haim, 5020000 Beit Dagan (IL); SHEPS, Tal, 5401453 Givat Shmuel (IL); MURPHY, Brian Patrick, Irvine, California 92614 (US); DOHERTY, Thomas V., Irvine, California 92614 (US)
(74) Representative: Eisenführ Speiser
(86) International application number: PCT/IB2022/053805
(87) International publication number: WO 2022/229815

(56) References cited:
- US-A1- 2006 020 336
- US-A1- 2006 178 700
- US-A1- 2006 282 161
- US-A1- 2007 080 188
- US-A1- 2011 029 071

## Description

### CROSS-REFERENCES TO RELATED APPLICATIONS

This application claims priority from US Provisional Application 63/181,565 to Hoffer et al., filed April 29, 2021, and entitled "Transcatheter devices and methods for treatment of a heart".

### BACKGROUND

A function of mammalian hearts is based on heart muscle contracting and creating pressure within ventricles of the heart, causing the blood to flow from the ventricles into arteries. The blood from the body returns to atria of the heart via veins. Valves, between each heart atrium and the corresponding heart ventricle, as well as between the heart ventricles and the corresponding arteries, inhibit backflow from occurring. The valves between the atria and the ventricles of the heart are known as the atrioventricular valves. At least in humans, the atrioventricular valve between the left atrium and the left ventricle is known as the mitral valve, and the atrioventricular valve between the right atrium and the right ventricle is known as the tricuspid valve. Each of these valves includes of a plurality of leaflets that coapt with each other when the valve is closed and have a space formed therebetween when the valve is open.

Proper closing of the mitral and tricuspid valves is critical for proper function of the heart, and many medical conditions, some life threatening, result from improper closing of the valves which creates backflow (regurgitation) from the ventricles into the corresponding atria. An annuloplasty procedure may be needed to reshape the annulus of a tricuspid or mitral valve that does not close properly.

US 2011/0029071 A1 (US'071) discloses a coronary sinus ring apparatus, including an elongated body for deployment in a coronary sinus, an aperture in a long side of the body, at least a partial lumen extending from one end of the elongated body to at least the aperture, and a guide within the elongated body positioned and shaped to guide an element inserted along the lumen to the aperture. US'071 also discloses an apparatus for treating a heart, including a delivery tube sized for and adapted for insertion into a body, a sharp tip adapted to be pushed through cardiac muscle, an elongate tensioning element, and a foldable anchor adapted to couple the elongate tension element to cardiac muscle tissue, wherein the delivery tube encloses one or both of the elongate tensioning element and the foldable anchor.

US 2006/0020336 A1 discloses an apparatus for reducing mitral regurgitation comprising: a plication assembly comprising a first anchoring element, a second anchoring element, and a linkage construct connecting the first anchoring element to the second anchoring element; and a catheter adapted to deliver the first anchoring element to the coronary sinus.

US 2006/0178700 A1 discloses a medical device which comprises a generally cylindrical treatment element for location between a pair of valve leaflets situated between an atrium and a ventricle of a heart.

### SUMMARY

The claimed invention is defined in independent claim 1 and relates to a system for use with a subject. Some preferred configurations of the claimed invention are defined in dependent claims 2 to 15. Also described herein are related aspects, examples, embodiments and arrangements useful for understanding the claimed invention, and which do not necessarily constitute embodiments of the claimed invention. The subject-matter for which protection is sought is defined by the claims.

This summary is meant to provide some examples and is not intended to be limiting of the scope of the invention in any way. For example, any feature included in an example of this summary is not required by the claims, unless the claims explicitly recite the features. Also, the features, components, steps, concepts, etc. described in examples in this summary and elsewhere in this disclosure can be combined in a variety of ways. Various features and steps as described elsewhere in this disclosure can be included in the examples summarized here.

In accordance with some applications herein, a tether is advanced into a coronary blood vessel or artery, and the two termini, or ends, of the tether are advanced through the wall of the coronary blood vessel or artery into an adjacent chamber of the heart, at two different locations. The termini are drawn out of the heart chamber (as described in more detail hereinbelow), such that, for each of the locations, a segment of the tether extends through the heart chamber, from a respective one of the locations. A tissue anchor is slid over and along at least one of the termini and is anchored to the tissue of the heart chamber, such that a part of the tether extends between the wall of the coronary blood vessel or artery and the tissue anchor. The heart chamber is then reshaped by modifying tension in the tether, e.g., in the part of the tether that extends between the coronary blood vessel or artery and the tissue anchor. The tension in the tether is then locked, to maintain the newly achieved shape of the heart chamber. For some applications, the chamber is an atrium of the heart, and reshaping the chamber advantageously reshapes and improves the function of the atrioventricular valve disposed between the atrium and the ventricle of the heart that is downstream of the atrium.

There is provided, in accordance with some applications, a method of repairing a heart valve of a heart of a subject. The method includes transluminally advancing a tether into a coronary blood vessel or artery of the heart of the subject. The coronary blood vessel or artery may at least partly circumscribe a heart chamber of the heart.

The method can include advancing a first terminus of the tether through a wall of the coronary blood vessel or artery into the heart chamber at first location. The first terminus of the tether can be transluminally drawn out of the heart chamber such that a first segment of the tether extends from the first location through the heart chamber.

The method can further include advancing a second terminus of the tether, through the wall of the coronary blood vessel or artery, into the heart chamber at a second location. The second terminus of the tether can be transluminally drawn out of the heart chamber such that a second segment of the tether extends from the second location through the heart chamber.

At least one tissue anchor can be slid over and along at least one of the first and second termini and can be anchored to tissue of the heart chamber. As such, a part of the tether extends between the at least one tissue anchor and the coronary blood vessel or artery.

The heart chamber can be reshaped by modifying tension of at least one part of the tether extending between the at least one tissue anchor and the coronary blood vessel or artery, and the tension of the tether can be locked, following the reshaping of the heart chamber.

In some applications, the sliding of the at least one anchor can include sliding one anchor over and along both the first and second termini. In some applications, the locking of the tension in the tether can include locking the tension adjacent the one anchor.

In some applications, the locking of the tension can include locking the first and second termini of the tether using a single lock.

In some applications, the sliding of the at least one anchor can include sliding a first tissue anchor over and along the first terminus and can include anchoring the first tissue anchor to tissue of the heart chamber at a first placement. As such, a first part of the tether extends between the first tissue anchor and the coronary blood vessel or artery.

In some applications, the sliding can further include sliding a second tissue anchor over and along the second terminus and can include anchoring the second tissue anchor to tissue of the heart chamber at a second placement. As such second part of the tether extends between the second tissue anchor and the coronary blood vessel or artery.

In some applications, the locking of the tension can include locking the first and second termini of the tether adjacent the first and second tissue anchors, respectively.

In some applications, the locking of the tension can include locking both the first and second termini of the tether using a single lock.

In some applications, the method can further include, prior to the locking of the tension, sliding the single lock onto the first and second termini of the tether, and along the tether, to a locking point.

In some applications, sliding of the single lock onto the first and second termini of the tether can occur outside the subject's body, and the sliding of the single lock along the tether can occur, at least partially, transluminally.

In some applications, modifying of tension in the tether can include locking one of the first and second termini adjacent a corresponding one of the first and second tissue anchors; and pulling the other one of the first and second termini.

In some applications, the locking of the tension can include locking the other one of the first and second termini adjacent a corresponding other one of the first and second tissue anchors.

In some applications, the modifying of the tension in the tether can include pulling on the first and second termini of the tether, and subsequently locking the first terminus of the tether adjacent the first tissue anchor and the second terminus of the tether adjacent the second tissue anchor.

In some applications, the heart chamber is a left atrium of the heart, and the coronary blood vessel or artery is a left coronary artery of the subject.

In some applications, the left coronary artery is a left circumflex artery of the subject.

In some applications, the reshaping of the heart chamber includes reshaping of a mitral valve of the subject.

In some applications, at least one of the transluminally drawing the first terminus and the transluminally drawing the second terminus includes drawing the first and the second termini through at least one of an interspatial septum of the heart and a vena cava of the subject.

In some applications, the vena cava is a superior vena cava.

In some applications, the vena cava is an inferior vena cava, and the drawing of at least one of the first terminus and the second terminus may further be through a femoral vein of the subject.

In some applications, the first and second termini are both drawn through one of the superior vena cava and the inferior vena cava.

In some applications, one of the first and second termini is drawn through the superior vena cava, and the other of the first and second termini is drawn through the inferior vena cava

In some applications, the advancing of the tether into the coronary blood vessel or artery is through a femoral artery and an aorta of the subject.

In some applications, the heart chamber is a right atrium of the heart, and the coronary blood vessel or artery is a right coronary artery of the subject.

In some applications, the reshaping of the heart chamber includes reshaping of a tricuspid valve of the subject.

In some applications, at least one of the transluminally drawing the first terminus and the transluminally drawing the second terminus includes drawing at least one of the first and the second termini through a vena cava of the subject.

In some applications, the vena cava is a superior vena cava.

In some applications, the vena cava is an inferior vena cava, and the drawing of at least one of the first terminus and the second terminus can further be through a femoral vein of the subject.

In some applications, the first and second termini are both drawn through one of the superior vena cava and the inferior vena cava.

In some applications, one of the first and second termini is drawn through the superior vena cava, and the other of the first and second termini is drawn through the inferior vena cava.

In some applications, the drawing of the first terminus and the drawing of the second terminus includes drawing the first and second termini out of the subject's body. In some applications, the sliding of the at least one anchor onto the at least one of the first and second termini of the tether occurs outside of the subject's body, and the sliding of the at least one anchor along the tether causes the at least one anchor to be delivered into the subject's body.

In some applications, the drawing of the first terminus and the drawing of the second terminus includes drawing the first and second termini out of the heart chamber within the subject's body. In some applications, the sliding of the at least one anchor onto the at least one of the first and second termini of the tether and along the tether occurs within the subject's body.

In some applications, at least one of the drawing of the first terminus and the drawing of the second terminus can be accomplished using a snare.

In some applications, the drawing of the first terminus and the drawing of the second terminus are each accomplished using a snare.

In some applications, the snare used for drawing the first terminus and the snare used for drawing of the second terminus are the same snare.

In some applications, the method can further include, prior to at least one of the drawing of the first terminus and the drawing of the second terminus, transluminally advancing the snare, out of a catheter, to the heart chamber.

In some applications, at least one of the drawing of the first terminus and the drawing of the second terminus can be through the catheter.

In some applications, the method can further include transluminally advancing a support tube defining a lumen into the coronary blood vessel or artery, the tether extending through the lumen of the support tube and being slidable relative to the support tube.

In some applications, the transluminally advancing of the support tube can be carried out prior to the advancing of the first terminus of the tether through the wall of the coronary blood vessel or artery into the heart chamber.

In some applications, the transluminally advancing of the support tube can be carried out following the advancing of the first terminus of the tether through the wall of the coronary blood vessel or artery into the heart chamber, and prior to the advancing of the second terminus of the tether through the wall of the coronary blood vessel or artery into the heart chamber.

The above method(s) can be performed on a living animal or on a simulation, such as on a cadaver, cadaver heart, simulator (e.g., with the body parts, heart, tissue, etc. being simulated), etc.

There is further provided, in accordance with claim 1, a system for use with a subject. The system includes a tether configured to be transluminally advanced into a coronary blood vessel or artery of the heart of the subject. The coronary blood vessel or artery at least partially transcribing a heart chamber of the heart. The tether has a first terminus and a second terminus, the first terminus configured to be advanced through a wall of the coronary blood vessel or artery into the heart chamber at a first location and the second terminus configured to be advanced through the wall of the coronary blood vessel or artery into the heart chamber at a second location.

As defined in claim 1, the system further includes at least one device configured to transluminally draw the first and second termini of the tether, from the first and second locations, respectively, out of the heart chamber. As such, a first segment of the tether extends from the first location through the heart chamber and a second segment of the tether extends from the second location through the heart chamber.

As defined in claim 1, the system further includes a support tube defining a lumen, the support tube being configured to be transluminally advanced into the coronary blood vessel or artery. The tether extends through the lumen of the support tube and being slidable relative to the support tube.

As defined in claim 1, the system further includes at least one tissue anchor, configured to be slid over and along at least one of the first and second termini, and to anchor the at least one of the first and second termini to tissue of the heart chamber, such that a part of the tether extends between the at least one tissue anchor and the coronary blood vessel or artery.

As defined in claim 1, the system further includes at least one lock, configured, following tensioning of the tether, to lock tension in the tether.

In some applications, the at least one tissue anchor can include a single tissue anchor, configured to be slid over and along both the first and second termini, and to anchor the first and second termini to tissue of the heart chamber, such that two parts of the tether extend between the tissue anchor and the coronary blood vessel or artery.

In some applications, the at least one lock can include a single lock configured to lock the first and second termini of the tether adjacent the single tissue anchor.

In some applications, the at least one tissue anchor can include a first tissue anchor configured to be slid over and along the first terminus and to anchor the first terminus to tissue of the heart chamber at a first placement, such that a first part of the tether extends between the first tissue anchor and the coronary blood vessel or artery. In some applications, a second tissue anchor can be configured to be slid over and along the second terminus and to anchor the second terminus to tissue of the heart chamber at a second placement, such that a second part of the tether extends between the second tissue anchor and the coronary blood vessel or artery.

In some applications, the at least one lock includes a single lock configured to be slid onto the first and second termini of the tether and to lock the first and second termini of the tether to a locking point adjacent one of the first and second tissue anchors.

In some applications, the at least one lock can include a first lock configured to be slid onto the first terminus of the tether and to lock the first terminus of the tether to a first locking point adjacent the first tissue anchor, and a second lock configured to be slid onto the second terminus of the tether and to lock the second terminus of the tether to a second locking point adjacent the second tissue anchor.

In some applications, the heart chamber is a left atrium of the heart, and the coronary blood vessel or artery is a left coronary artery of the subject.

In some applications, the left coronary artery is a left circumflex artery of the subject.

In some applications, the tension in the tether is configured to reshape a mitral valve of the subject.

In some applications, the at least one device is configured to draw at least one of the first and second termini through at least one of an interspatial septum of the heart and a vena cava of the subject.

In some applications, the vena cava is a superior vena cava.

In some applications, the vena cava is an inferior vena cava, and the at least one device can be configured to draw at least one of the first terminus and the second terminus further through a femoral vein of the subject.

In some applications, the at least one device is configured to draw the first and second termini through one of the superior vena cava and the inferior vena cava.

In some applications, the at least one device includes a first device configured to draw one of the first and second termini through the superior vena cava, and a second device configured to draw the other of the first and second termini through the inferior vena cava

In some applications, the tether is configured to be advanced into the coronary blood vessel or artery through a femoral artery and an aorta of the subject.

In some applications, the heart chamber is a right atrium of the heart, and the coronary blood vessel or artery is a right coronary artery of the subject.

In some applications, the tension in the tether is configured to reshape a tricuspid valve of the subject.

In some applications, the at least one device is configured to draw at least one of the first and second termini through a vena cava of the subject.

In some applications, the vena cava is a superior vena cava.

In some applications, the vena cava is an inferior vena cava, and the at least one device can be configured to draw at least one of the first and second termini further through a femoral vein of the subject.

In some applications, the at least one device is configured to draw the first and second termini through one of the superior vena cava and the inferior vena cava.

In some applications, the at least one device includes a first device configured to draw one of the first and second termini through the superior vena cava, and a second device configured to draw the other of the first and second termini through the inferior vena cava.

In some applications, the system can further include a first longitudinal catheter configured to be transluminally advanced to the coronary blood vessel or artery. The tether can be configured to be advanced to the coronary blood vessel or artery distally out of the first catheter.

In some applications, the at least one device includes at least one snare.

In some applications, the at least one snare includes a single snare configured to draw the first and second termini.

In some applications, the at least one snare includes a first snare configured to draw the first terminus and a second snare configured to draw the second terminus.

In some applications, the at least one snare is configured to be transluminally advanced out of a catheter, to the heart chamber, prior to at least one of the drawing of the first terminus and the drawing of the second terminus.

In some applications, the system can further include at least one second catheter. The at least one device can be configured to draw the first and second termini through the at least one second catheter.

In some applications, the support tube is configured to be advanced into the coronary blood vessel or artery distally out of the first catheter.

In accordance with a second aspect of the teachings herein, methods and devices are provided to protect a coronary blood vessel or artery of the heart of a subject during a medical procedure in the heart of the subject. A compressed structure, comprising a radiopaque material, is expanded within the coronary blood vessel or artery into an elongate structure. The elongate structure is visible using fluoroscopic imaging. The medical procedure can include anchoring of a tissue anchor within the heart chamber adjacent the coronary blood vessel or artery in which the elongate structure is disposed. The heart is imaged using fluoroscopic tools, such that the elongate structure is visible. The location for anchoring of the tissue anchor is selected, based on the obtained image(s), to avoid damage to the coronary blood vessel or artery during the anchoring. For some applications, the elongate structure is retained in the coronary blood vessel or artery while the anchoring is performed, and the anchoring can be performed under real-time fluoroscopic guidance, aided by the visibility of the elongate structure.

In some cases, the elongate structure comprises an electrically conductive material, and an electrical signal (e.g., a voltage) is applied, by a control subsystem, between the elongate structure and the anchor and/or an anchor driver that is delivering the anchor. Proximity or contact between the tissue anchor and the elongate structure is detected by the control subsystem (e.g., by detecting the electrical signal), and a signal such as a visual, audible, or haptic signal is provided to indicate the proximity and/or contact between the anchor and the elongate structure. For some applications, a different elongate structure (e.g., an elongate structure that is not expandable and/or does not comprise a radiopaque material) is used in a similar manner.

There is further provided, in accordance with some applications, a method of avoiding damage to a coronary blood vessel or artery of a heart of a subject during a medical procedure, the coronary blood vessel or artery being adjacent to a heart chamber of the heart. The method includes, within the coronary blood vessel or artery, expanding a compressed elongate tube that includes a radiopaque material.

The method can further include anchoring a tissue anchor within the heart chamber adjacent the coronary blood vessel or artery.

In some applications, placement of the tissue anchor during the anchoring can be selected, based on at least one fluoroscopic image that includes the elongate tube, to avoid damaging the coronary blood vessel or artery during the anchoring.

In some applications, the method can further include, prior to the expanding, transluminally advancing the compressed elongate tube to the coronary blood vessel or artery.

In some applications, the method can further include, following the anchoring of the tissue anchor, transluminally removing the elongate tube from the coronary blood vessel or artery.

In some applications, the transluminally removing can include recompressing the elongate tube, and transluminally removing the compressed elongate tube from the coronary blood vessel or artery.

In some applications, the heart chamber is a left atrium of the heart, and the coronary blood vessel or artery is a left coronary artery of the subject.

In some applications, the left coronary artery is a left circumflex artery of the subject.

In some applications, the anchoring is into an annulus of a mitral valve of the subject.

In some applications, the elongate tube is sufficiently long to extend around at least majority of the annulus of the mitral valve.

In some applications, the advancing of the compressed elongate tube into the coronary blood vessel or artery is through a femoral artery and an aorta of the subject.

In some applications, the heart chamber is a right atrium of the heart, and the coronary blood vessel or artery is a right coronary artery of the subject.

In some applications, the anchoring is into an annulus of a tricuspid valve of the subject.

In some applications, the elongate tube is sufficiently long to extend around at least majority of the annulus of the tricuspid valve.

In some applications, the advancing of the compressed elongate tube into the coronary blood vessel or artery is through a coronary ostia at an aortic root of the subject.

In some applications, the advancing includes advancing the compressed elongate tube transluminally over a guidewire.

In some applications, the elongate tube includes a metal frame or a mesh frame.

In some applications, the expanding can include retracting an external sheath from the compressed elongate tube, thereby allowing the elongate tube to expand.

In some applications, the expanding can include pulling one end of the elongate tube away from the opposing end of the elongate tube.

In some applications, the method can further include, prior to the expanding, transluminally advancing a pulling element to the distal end of the elongate tube, within the coronary blood vessel or artery.

In some applications, during the anchoring, the body of the subject is devoid of contrast agents.

In some applications, the elongate tube is made of an electrically conductive material. In some applications, an anchor driver can be mechanically and electrically coupled to the tissue anchor, is electrically coupled to a control subsystem such that contact between the anchor and the elongate tube generates a detectable signal.

In some applications, the method can further include, during the anchoring, monitoring for a detectable signal indicative of contact between the tissue anchor and the elongate tube.

In some applications, the method can further include, in response to identification of the detectable signal, repositioning the tissue anchor in another location.

The above method(s) can be performed on a living animal or on a simulation, such as on a cadaver, cadaver heart, simulator (e.g., with the body parts, heart, tissue, etc. being simulated), etc.

There is further provided, in accordance with some applications, a system for use with a subject. The system can include an elongate tube including a radiopaque and electrically conductive material. The elongate tube can be configured to be transluminally advanced into a coronary blood vessel or artery in a heart of the subject, in a compressed state, and expanded within the coronary blood vessel or artery.

The system can further include a tissue anchor configured to be anchored within a heart chamber of the subject, adjacent the coronary blood vessel or artery.

The system can further include at least one fluoroscopic image capturing device, configured to capture at least one fluoroscopic image during anchoring of the tissue anchor within the heart chamber. In some applications, the at least one fluoroscopic image includes the elongate tube.

The system can further include an anchor driver, which can be mechanically and electrically coupled to the tissue anchor.

A control subsystem can be configured to generate a detectable signal when the tissue anchor contacts the elongate tube.

In some applications, the at least one fluoroscopic image is configured to assist an operator in avoiding damaging the coronary blood vessel or artery during anchoring of the tissue anchor.

In some applications, the elongate tube is a compressible elongate tube having a compressed operative orientation and an expanded operative orientation. In some applications, the elongate tube can be configured to be advanced into the coronary blood vessel or artery when in the compressed operative orientation.

In some applications, the elongate tube can further be configured to be transluminally removed from the coronary blood vessel or artery, following the anchoring of the tissue anchor.

In some applications, the heart chamber is a left atrium of the heart, and the coronary blood vessel or artery is a left coronary artery of the subject.

In some applications, the left coronary artery is a left circumflex artery of the subject.

In some applications, the tissue anchor can be configured to be anchored into an annulus of a mitral valve of the subject.

In some applications, the elongate tube is sufficiently long to extend around at least majority of the annulus of the mitral valve.

In some applications, the elongate tube is configured to be advanced into the coronary blood vessel or artery is through a femoral artery and an aorta of the subject.

In some applications, the heart chamber is a right atrium of the heart, and the coronary blood vessel or artery is a right coronary artery of the subject.

In some applications, the tissue anchor is configured to be anchored into an annulus of a tricuspid valve of the subject.

In some applications, the elongate tube is sufficiently long to extend around at least majority of the annulus of the tricuspid valve.

In some applications, the elongate tube is configured to be advanced into the coronary blood vessel or artery is through a coronary ostia at an aortic root of the subject.

In some applications, the system further includes a guidewire. In some applications, the compressed elongate tube is configured to be transluminally advanced into the coronary blood vessel or artery over the guidewire.

In some applications, the elongate tube includes a metal frame or a mesh frame.

In some applications, the elongate tube can further include an external sheath adapted to hold the elongate tube in the compressed operative orientation.

In some applications, the system can further include a pulling element configured to pull a distal end of the elongate tube distally with respect to a proximal end of the elongate tube, when the elongate tube is in the compressed operative orientation, thereby to expand the elongate tube.

There is further provided, in accordance with some applications, a method of avoiding damage to a coronary blood vessel or artery of a heart of a subject during a medical procedure, the coronary blood vessel or artery being adjacent to a heart chamber of the heart. The method includes advancing an elongate structural element into the coronary blood vessel or artery, the elongate structural element being configured to emit an electrical signal.

The method can further include causing elongate structural element to emit an electrical signal within the coronary blood vessel or artery, and can include, using an anchor driver driving a tissue anchor, detecting the electrical signal adjacent the anchor driver.

In some applications, the method can further include anchoring the tissue anchor within the heart chamber adjacent the coronary blood vessel or artery, at an anchoring position at which the electrical signal detected by the anchor driver is below a predetermined threshold, thereby to avoid damaging the coronary blood vessel or artery during the anchoring.

In some applications, the anchoring includes anchoring the tissue anchor at an anchoring position at which the electrical signal detected by the anchor driver is an optimal electrical signal.

In some applications, the elongate structural element includes an elongate wire.

In some applications, the elongate structural element includes an elongate tube.

In some applications, the method can further include transluminally advancing the elongate tube, in a compressed position, to the coronary blood vessel or artery, and expanding the elongate tube in the coronary blood vessel or artery.

In some applications, the method can further include, following the anchoring of the tissue anchor, transluminally removing the elongate structural element from the coronary blood vessel or artery.

In some applications, the heart chamber is a left atrium of the heart, and the coronary blood vessel or artery is a left coronary artery of the subject.

In some applications, the left coronary artery is a left circumflex artery of the subject.

In some applications, the anchoring is into an annulus of a mitral valve of the subject.

In some applications, the elongate structural element is sufficiently long to extend around at least majority of the annulus of the mitral valve.

In some applications, the advancing of elongate structural element into the coronary blood vessel or artery is through a femoral artery and an aorta of the subject.

In some applications, the heart chamber is a right atrium of the heart, and the coronary blood vessel or artery is a right coronary artery of the subject.

In some applications, the anchoring is into an annulus of a tricuspid valve of the subject.

In some applications, the elongate structural element is sufficiently long to extend around at least majority of the annulus of the tricuspid valve.

In some applications, the advancing of the elongate structural element into the coronary blood vessel or artery is through a coronary ostia at an aortic root of the subject.

In some applications, during the anchoring, the body of the subject is devoid of contrast agents.

The above method(s) can be performed on a living animal or on a simulation, such as on a cadaver, cadaver heart, simulator (e.g., with the body parts, heart, tissue, etc. being simulated), etc.

There is further provided, in accordance with some applications, a system for use with a subject, the system including an elongate structural element formed of an electrical signal emitting material. In some applications, the elongate structure is configured to be transluminally advanced into a coronary blood vessel or artery in a heart of the subject and to emit an electrical signal within the coronary blood vessel or artery.

The system can further include a tissue anchor configured to be anchored within a heart chamber of the subject, adjacent the coronary blood vessel or artery.

In some applications, the system further includes an anchor driver, which can be mechanically and electrically coupled to the tissue anchor, the anchor driver being configured to detect an electrical signal adjacent the anchor driver.

In some applications, the anchor driver is configured to anchor the tissue anchor to tissue of the heart chamber at an anchoring position at which the electrical signal detected by the anchor driver is below a predetermined threshold, thereby to avoid damaging the coronary blood vessel or artery during anchoring of the tissue anchor.

In some applications, the anchor driver is configured to anchor the tissue anchor at an anchoring position at which the electrical signal detected by the anchor driver is a minimum electrical signal.

In some applications, the elongate structural element includes an elongate wire.

In some applications, the elongate structural element includes an elongate tube.

In some applications, the elongate tube is configured to be transluminally advanced into the coronary blood vessel or artery in a compressed position, and then expanded within the coronary blood vessel or artery.

In some applications, the heart chamber is a left atrium of the heart, and the coronary blood vessel or artery is a left coronary artery of the subject.

In some applications, the left coronary artery is a left circumflex artery of the subject.

In some applications, the tissue anchor is configured to be anchored into an annulus of a mitral valve of the subject.

In some applications, the elongate structural element is sufficiently long to extend around at least majority of the annulus of the mitral valve.

In some applications, the elongate structural element is configured to be advanced into the coronary blood vessel or artery through a femoral artery and an aorta of the subject.

In some applications, the heart chamber is a right atrium of the heart, and the coronary blood vessel or artery is a right coronary artery of the subject.

In some applications, the tissue anchor is configured to be anchored into an annulus of a tricuspid valve of the subject.

In some applications, the elongate structural element is sufficiently long to extend around at least majority of the annulus of the tricuspid valve.

In some applications, the elongate structural element is configured to be advanced into the coronary blood vessel or artery through a coronary ostia at an aortic root of the subject.

### BRIEF DESCRIPTION OF THE FIGURES

The foregoing discussion will be understood more readily from the following detailed description, when taken in conjunction with the accompanying Figures, in which:
Fig. 1 is a schematic illustration of an example device according to an aspect of the teachings herein being delivered through the aorta of a subject into a left coronary artery of the heart of the subject, according to an example method;
Figs. 2A to 2L are schematic sectional view illustrations of steps of an example method of treatment of a mitral valve of the heart of the subject;
Figs. 3A and 3B are schematic sectional view illustrations of example methods, which can be similar or include similarities to the method of Figs. 2A to 2L;
Fig. 4 is a schematic illustration of an example device according to an aspect of the teachings herein being delivered through the aorta of a subject into a right coronary artery of the heart of the subject, according to an example method;
Figs. 5A to 5L are schematic sectional view illustrations of steps of an example method of treatment of a tricuspid valve of the heart of the subject;
Figs. 6A and 6B are schematic sectional view illustrations of example methods, which can be similar or include similarities to the method of Figs. 5A to 5L;
Fig. 7 is a schematic illustration of an example device according to an aspect of the teachings herein being delivered through the aorta of a subject into a left coronary artery of the heart of the subject, according to an example method;
Figs. 8A to 8K are schematic sectional view illustrations of steps of an example method of preventing damage to the coronary blood vessel or coronary artery during treatment of a mitral valve of the heart of the subject;
Figs. 9A to 9C are schematic sectional view illustrations of steps of an example method of preventing damage to the coronary blood vessel or coronary artery during treatment of a mitral valve of the heart of the subject; and
Figs. 10A to 10C are schematic sectional view illustrations of steps of an example method of preventing damage to the coronary blood vessel or coronary artery during treatment of a mitral valve of the heart of the subject.

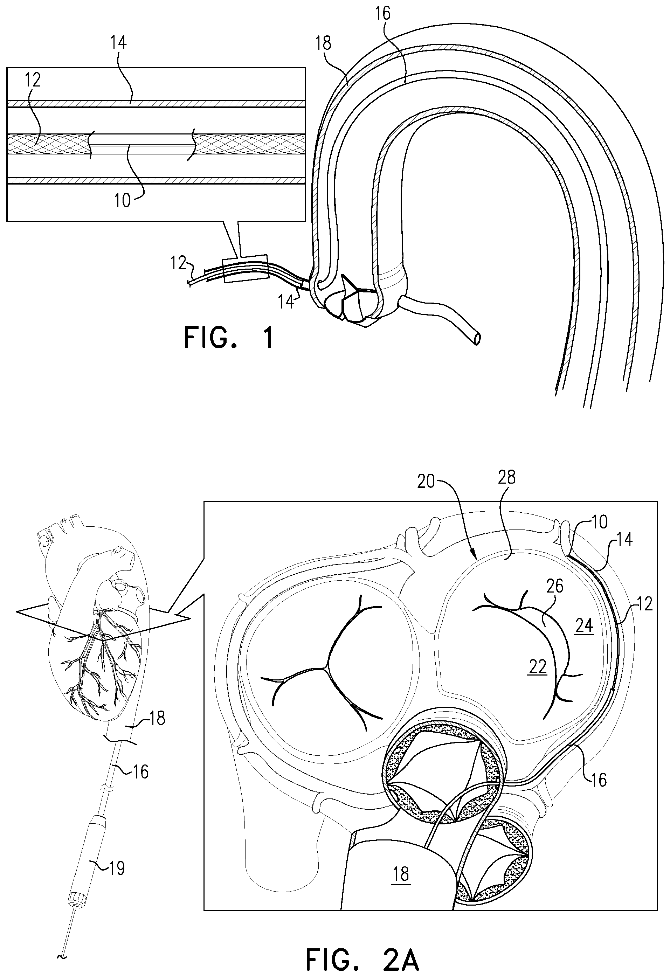

The invention relates to a system as illustrated in fig. 1-6.

It will be appreciated by persons skilled in the art that the present invention is not limited to what has been particularly shown and described hereinabove. Rather, the scope of the present invention is defined by the appended claims.

### DETAILED DESCRIPTION

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure. Additionally, in order to avoid undue clutter from having too many reference numbers and lead lines on a particular drawing, some elements may not be explicitly identified in every drawing that contains that element.

It is to be understood that the scope of the invention is not limited in its application to the details of construction and the arrangement of the components set forth in the following description or illustrated in the drawings. The scope of the invention is defined by the appended claims. Furthermore, it is to be understood that the phraseology and terminology employed in the disclosure is for the purpose of description and should not be regarded as limiting.

For the purposes of this application, the term "subject" relates to any mammal, particularly humans.

Referring now to the drawings, Fig. 1 is a schematic illustration of an example device according to an aspect of the teachings herein being delivered through the aorta of a subject into a coronary blood vessel, in this example a left coronary artery (although various other blood vessels are also possible), of the heart of the subject, according to an example method. The method can be performed on a living animal or on a simulation, such as on a cadaver, cadaver heart, simulator (e.g., with the body parts, heart, tissue, etc. being simulated), etc.

As seen in Figure 1, a device according to the teachings herein includes a tether 10, which is surrounded by a support tube 12 and is slidable relative to the support tube. Tether 10 can be formed of any suitable material, such as metal, polymer, biomaterial wire, ribbon, or cord. Support tube 12 is typically flexible.

For treatment of the mitral valve (see reference numeral 20 in Figs. 2A-2L) of the subject, which is located between the left atrium and the left ventricle of the heart, tether 10 and support tube 12 are transluminally advanced into a coronary blood vessel, in this example a left coronary artery 14, which at least partially circumscribes the mitral valve (see for example Fig. 2A). For some applications, and as shown in Fig. 1, a catheter 16 delivers tether 10 and support tube 12 via aorta 18 (e.g., transfemorally) and into left coronary artery 14. In some applications, the left coronary artery may be or include the left circumflex artery of the heart of the subject.

In the illustrated example, a distal part of catheter 16 is advanced to the left coronary artery. Catheter 16 also has an extracorporeal proximal part 19 that can comprise a handle (e.g., as shown in Fig. 2A). The distal part of catheter 16 is guidable to the anatomical site, such as by being actively steerable (e.g., by being operatively coupled by one or more pullwires to proximal part 19, such as to a steering controller thereof), or by being passively guided and/or steered (e.g., by extending over or through another steerable element, such as an actively steerable catheter).

Reference is now made to Figs. 2A to 2L, which are schematic sectional view illustrations of steps of a method of treatment of mitral valve 20 of the heart of the subject using the device of Fig. 1, in accordance with some applications of the teachings herein. For example, the treatment of the mitral valve can be or include annuloplasty of the mitral valve. The method can be performed on a living animal or on a simulation, such as on a cadaver, cadaver heart, simulator (e.g., with the body parts, heart, tissue, etc. being simulated), etc.

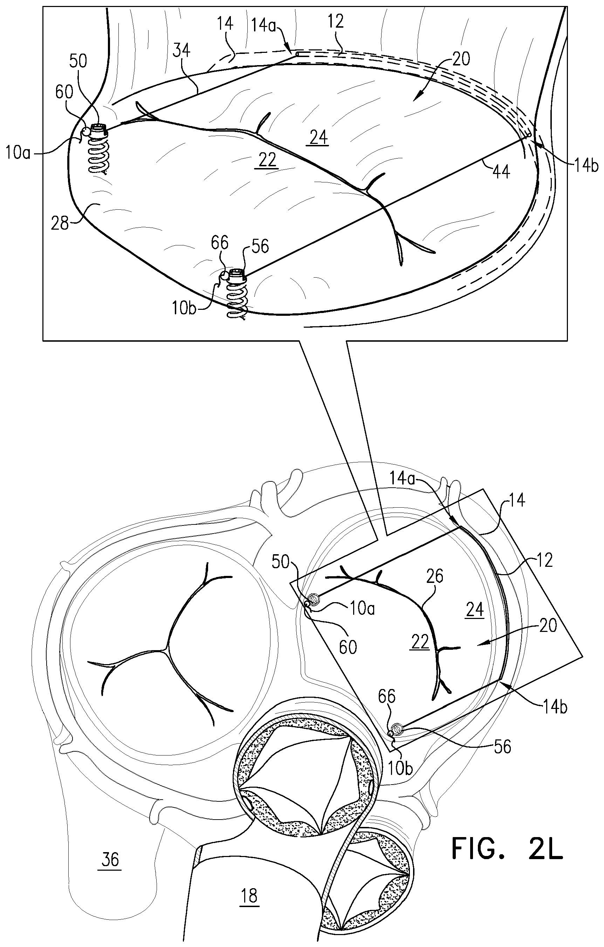

As seen in Fig. 2A, catheter 16 is used to advance tether 10, and typically support tube 12, via aorta 18 into left coronary artery 14, as described hereinabove with respect to Fig. 1. As seen in the sectional portion of Fig. 2A, left coronary artery 14 at least partially circumscribes the mitral valve 20 of the subject's heart (e.g., is disposed alongside an annulus 28 of the mitral valve). As seen, leaflets 22 and 24 of the mitral valve do not fully coapt, such that a gap 26 is formed therebetween. The treatment (e.g., annuloplasty) procedure described herein reshapes the mitral valve 20 (e.g., annulus 28), such that the leaflets 22 and 24 coapt and gap 26 is reduced or eliminated (see the end result in Fig. 2L).

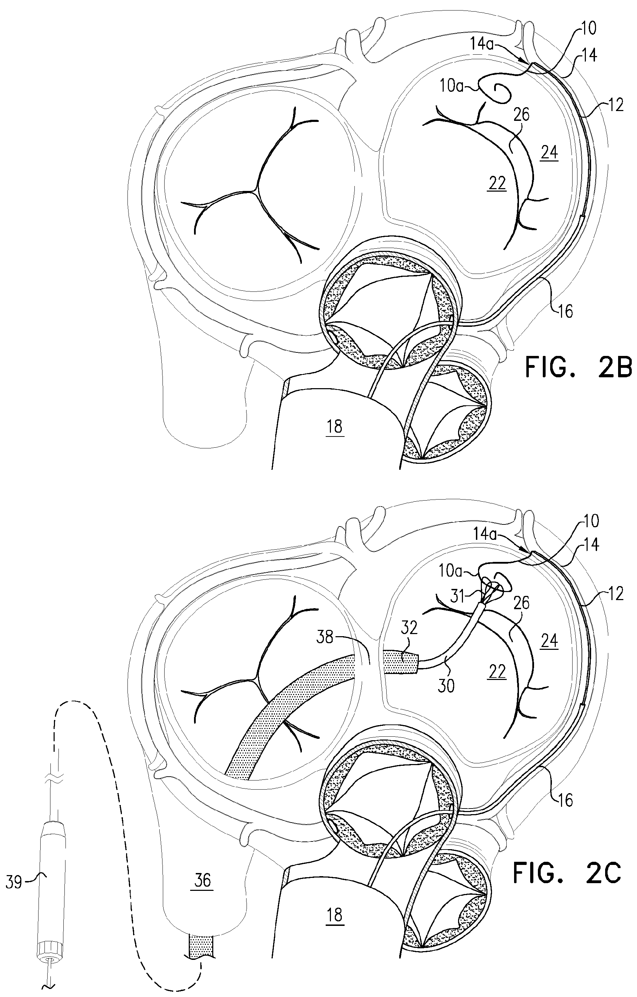

Turning to Fig. 2B, a hole is punctured (e.g., using a hollow needle) through the wall of the left coronary artery into the left atrium of the heart at a first puncture location 14a, and a first terminus 10a of tether 10 is advanced into the atrium.

In some applications, tether 10 is advanced into left coronary artery 14 as shown in Fig. 2A, without support tube 12. In some applications, following advancement of first terminus 10a of tether 10 into the left atrium, support tube 12 can be advanced onto, and around, the tether.

As seen in Fig. 2C, a snare tool 30, having a snare 31 at a distal part thereof, is transluminally introduced into the left atrium of the heart, and engages first terminus 10a of tether 10. In some applications, snare tool 30 is transluminally advanced into the left atrium using a second catheter 32. For example, snare tool 30 can be a component of second catheter 32 or can be a discrete device that is introduced via second catheter 32.

In some applications, and as shown, snare tool 30 is introduced into the left atrium through an inferior vena cava 36 (e.g., transfemorally) and through an opening in the interspatial septum 38 of the heart of the subject. In some applications, introduction of the snare tool can be via the superior vena cava.

In the illustrated example, a distal part of catheter 32 is advanced through the vena cava. Catheter 32 also has an extracorporeal proximal part 39 (e.g., as shown in Fig. 2C). The distal part of catheter 32 is guidable to the anatomical site, such as by being actively steerable (e.g., by being operatively coupled by one or more pullwires to proximal part 39, such as to a steering controller thereof), or by being passively guided and/or steered (e.g., by extending over or through another steerable element, such as an actively steerable catheter).

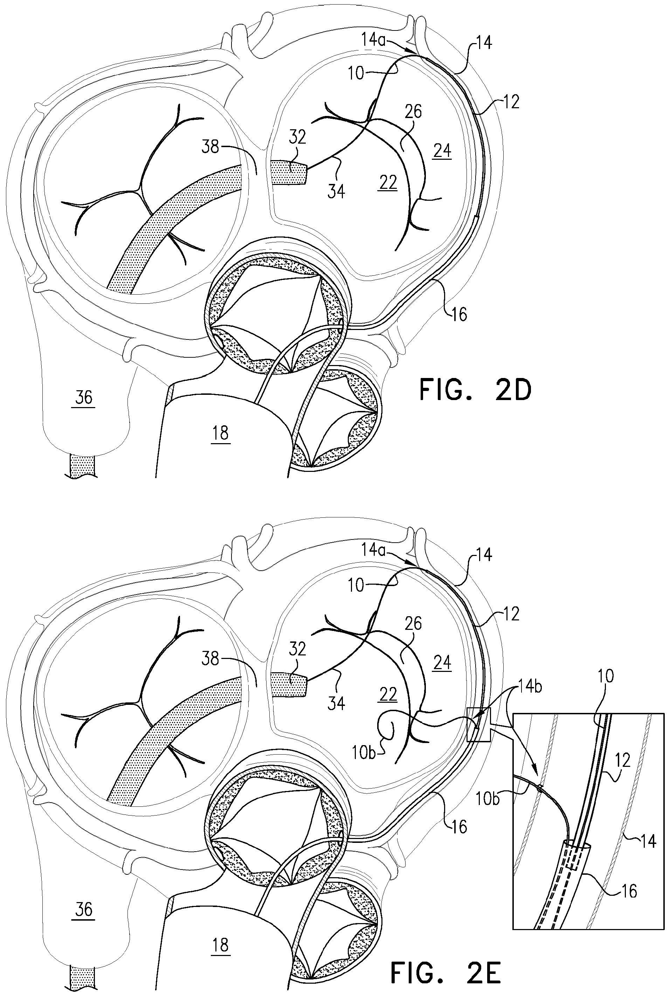

Snare 31 is then used to draw first terminus 10a of the tether out of the left atrium, e.g., as described in more detail hereinbelow, such that a first segment 34 of the tether extends from puncture location 14a across the left atrium, as seen in Fig. 2D. In some applications, first terminus 10a is drawn through catheter 32 (e.g., by withdrawing snare tool 30 through the catheter), as shown in Fig. 2D.

In some applications, and as shown, snare 31 draws first terminus 10a from the left atrium through the opening in the interspatial septum 38 and through inferior vena cava 36 of the subject. In some applications, withdrawal can be via the superior vena cava.

As explained in further detail hereinbelow, in some applications of the step shown in Fig. 2D, first terminus 10a of tether 10 is drawn fully out of the subject's body. In some applications of the step shown in Fig. 2D, first terminus 10a is drawn out of the left atrium, but remains within the subject's body.

Turning to Fig. 2E, a second hole is punctured through the wall of the left coronary artery into the left atrium of the heart at a second location 14b, and a second terminus 10b of the tether is advanced into the atrium. As seen, support tube 12 can be disposed between first location 14a and second location 14b - e.g., can extend between the first and second locations, terminating at each of the first and second locations.

In some applications, such as the example illustrated in Figs. 2A to 2L, first location 14a and second location 14b are selected to be near ends of gap 26 between leaflets 22 and 24.

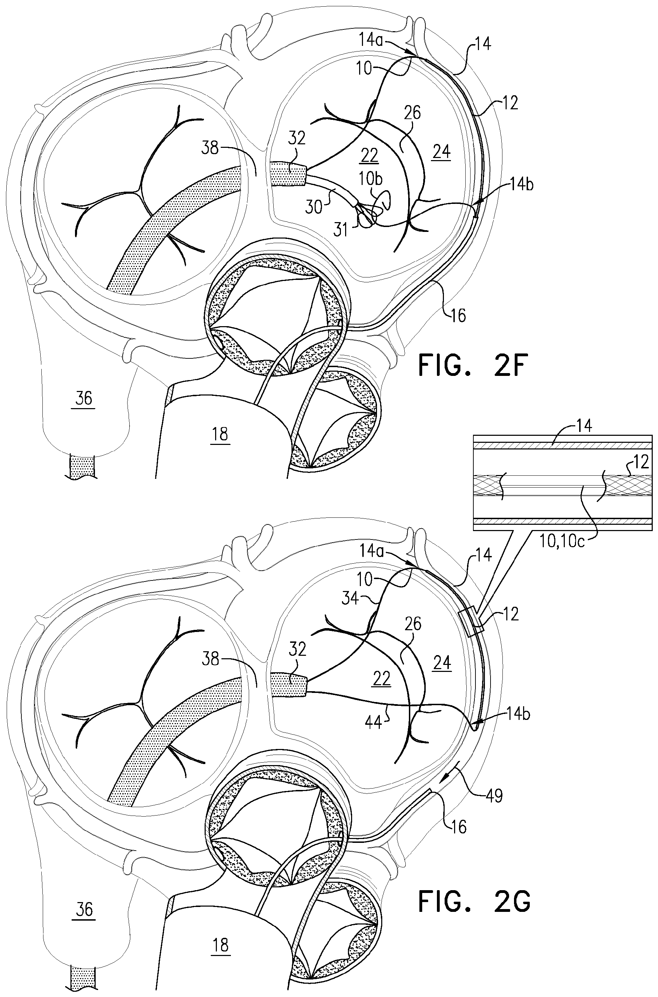

As seen in Fig. 2F, a snare tool, which can be snare tool 30 of Fig. 2C or can be a second snare tool, is transluminally introduced into the left atrium of the heart and engages second terminus 10b of the tether. The snare tool can be transluminally advanced into the left atrium via second catheter 32 (e.g., as described with respect to Fig. 2C, mutatis mutandis), or out of another, third, catheter.

Snare 31 of snare tool 30 (or another snare of another snare tool) is then used to draw second terminus 10b of the tether out of the left atrium, such that a second segment 44 of the tether extends from second puncture location 14b across the left atrium, as seen in Fig. 2G.

In some applications, and as shown, snare 31 of snare tool 30 (or another snare of another snare tool) draws second terminus 10b from the left atrium through the opening in interspatial septum 38 of the heart and through inferior vena cava 36 of the subject. In some applications, withdrawal can be via the superior vena cava.

In some applications, for example when the same snare and catheter are used for drawing both the first and second termini of tether 10, the first and second termini are both drawn from the heart chamber, or the left atrium, via the same route, i.e., via the same one of the superior and inferior vena cava. As shown in Fig. 2G, for some applications, tether 10 forms a loop from catheter 32, across the left atrium, into and along left coronary artery 14, back through the atrium, and into the catheter. This loop includes (i) segment 34 extending from catheter 32 across the atrium to location 14a, (ii) a bight 10c of tether 10 extending between location 14a and location 14b (e.g., within support tube 12), and (iii) segment 44 extending from location 14b back through the atrium and into the catheter.

In some applications, for example when snare tool 30 is used to draw first terminus 10a through catheter 32, and a separate snare is used to draw second terminus 10b through a separate catheter, each of the termini of tether 10 can be drawn out of the left atrium using a different route. For example, first terminus 10a may be drawn through the superior vena cava, while second terminus 10b may be drawn through the inferior vena cava.

As explained in further detail hereinbelow, in some applications of the step shown in Fig. 2G, second terminus 10b of tether 10 is drawn fully out of the subject's body. In some applications of the step shown in Fig. 2G, second terminus 10b is drawn out of the left atrium but remains within the subject's body. In some applications, the first and second termini of tether 10 are drawn out of the left atrium to the same extent (i.e., both are pulled fully out of the subject's body, or both are pulled out of the left atrium but remain in the subject's body).

In some applications, once both termini of tether 10 are drawn from the left atrium, catheter 16 can be removed from left coronary artery 14, for example by retraction of the catheter in the direction of arrow 49, through aorta 18.

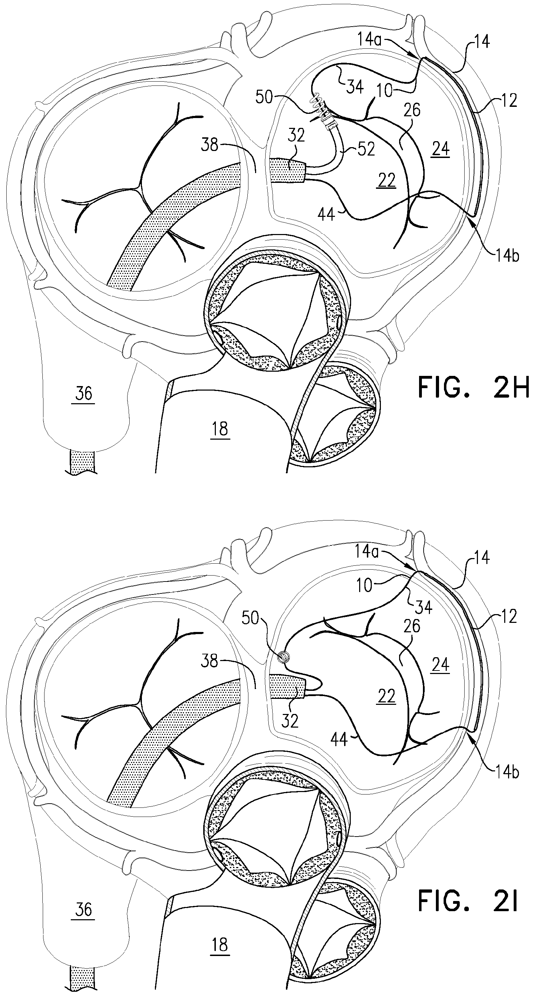

Turning to Fig. 2H, a first tissue anchor 50, e.g., driven by a driving tool 52, is slid over and along first terminus 10a toward first segment 34. First tissue anchor 50 is anchored into tissue of the left atrium, for example into annulus 28, such that first segment 34 now extends between left coronary artery 14 and first tissue anchor 50, as seen in Fig. 2I. First tissue anchor 50 can be anchored across mitral valve 20 from left coronary artery 14 (e.g., from support tube 12), such as at an anterior region of the left atrium, e.g., in proximity to the root of anterior leaflet 22 or a commissure at which the anterior leaflet meets posterior leaflet 24. At this stage, first segment 34 may be slack.

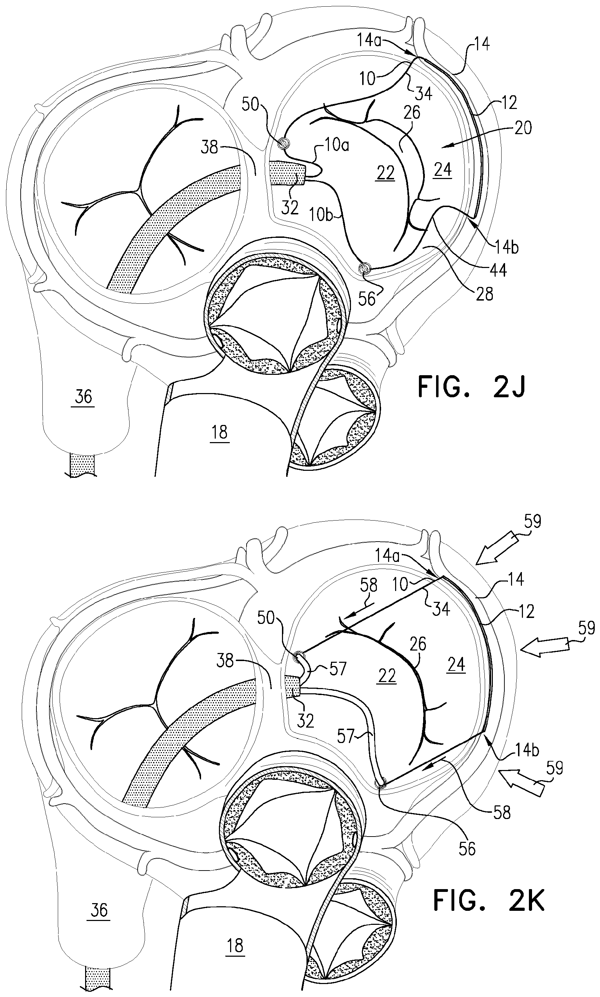

As seen in Fig. 2J, a second tissue anchor 56 is slid over and along second terminus 10b toward second segment 44. Second tissue anchor 56 is anchored into tissue of the left atrium, for example into annulus 28, such that second segment 44 now extends between left coronary artery 14 and the second tissue anchor. Second tissue anchor 56 can be anchored across mitral valve 20 from left coronary artery 14 (e.g., from support tube 12), such as at an anterior region of the left atrium, e.g., in proximity to the root of anterior leaflet 22 or a commissure at which the anterior leaflet meets posterior leaflet 24. At this stage, second segment 44 may be slack. Second tissue anchor 56 can be driven by driving tool 52, or by another anchor driving tool.

Each of anchors 50 and 56 can include a head that is slidably couplable to tether 10, e.g., by the head comprising an eyelet that is threadable onto the tether. Each of anchors 50 and 56 can include a tissue-engaging element, which can be a helical screw-in tissue-engaging element (as described herein) or can be another type of tissue-engaging element, such as a dart or a staple. For some applications, each of anchors 50 and 56 can comprise an anchor described in one or more of the following:
- US Patent Application 17/145,258 to Kasher et al., filed January 8, 2021, which published as US 2021/0145584;
- US Provisional Patent Application 63/147,699 to Shafigh et al., filed February 9, 2021; and
- International Patent Application PCT/IB2022/051099 to Shafigh et al., filed February 8, 2022.

Driving tool 52 can be advanced via second catheter 32 (e.g., as shown in Fig. 2H), or via another catheter. In some applications, and as shown, driving tool 52 is rotatable, and anchors the tissue anchor(s) by screwing the tissue anchor(s) into the tissue of annulus 28. Following anchoring of tissue anchors 50 and 56, driving tool 52 (and, if one is used, another driving tool) can be retracted from the left atrium, for example via second catheter 32.

Turning to Fig. 2K, the tension in tether 10 is modified (e.g., tension is applied to the tether). In some applications, this is done by pulling on one or both of termini 10a and 10b. This pulling is indicated by arrows 58. Tensioning may be facilitated by one or more transluminally introduced tools 57 that can, for example, provide an opposing force against anchors 50 and 56. The application of tension to tether 10 may pull support tube 12 against the wall of left coronary artery 14 that is closest to the atrium (e.g., such that the tube presses against the annulus and/or atrium).

Modification of the tension in tether 10 causes reshaping of the mitral valve 20, for example in the direction of arrows 59, so as to close the gap 26. As shown in Figs. 2K-L, the reshaping of the mitral valve improves coaptation between leaflets 22 and 24 of the valve, thereby reducing (e.g., closing) gap 26.

In some applications, support tube 12 is designed and configured to distribute force, applied by tether 10, across the wall of left coronary artery 14, so as to prevent the tether from damaging the wall of the left coronary artery and/or of the wall of the atrium. In some applications, support tube 12 maintains puncture locations 14a and 14b at a fixed distance from one another, for example by preventing tether 10 from cutting (e.g., in a similar manner to a cheese wire) through the wall of the left coronary artery from one of the locations toward the other.

Following modification to the tension of the tether and reshaping of the valve, the tension in the tether is locked. In the example illustrated in Fig. 2L, a first lock 60 (which can optionally be referred to as a stopper) is slid along first terminus 10a of tether 10 and is locked to the tether, often in proximity to first anchor 50. A second lock 66 is slid along second terminus 10b of tether 10, and is locked to the tether, often in proximity to second anchor 56. Locks 60 and 66 can lock the tension in tether 10 by inhibiting the tether (e.g., termini 10a and 10b) from sliding past anchors 50 and 56, e.g., by the locks abutting the anchors. For some applications, lock 60 has one or more features described in US Patent Application 16/534,875 to Brauon et al., filed August 7, 2019, which published as US 2020/0015971.

In some applications, excess tether is then cut and removed from the left atrium, for example by retraction of catheter 32. The advancement of locks 60 and 66, and the cutting of excess tether, can be performed using tools 57.

In some applications, the modification of the tension in tether 10 can be accomplished by pulling on both termini 10a and 10b. In some applications, the modification of the tension in tether 10 can be accomplished by initially locking one of termini 10a and 10b adjacent the respective one of tissue anchors 50 and 56, and subsequently pulling on the other one of termini 10a and 10b to modify the tension in the tether. Once the desired tension of the tether is achieved, the other of termini 10a and 10b is locked adjacent the respective one of tissue anchors 50 and 56. For example, first terminus 10a can be locked adjacent tissue anchor 50, following which an end user or physician can pull on terminus 10b to change the tension in the tether, and then locks second terminus 10b adjacent second tissue anchor 56.

In applications in which the termini 10a and 10b of the tether are drawn out of the user's body, as described hereinabove, the tissue anchors 50 and 56, as well as locks 60 and 66, can be slid onto the termini of the tether extracorporeally. In some applications, in which the termini 10a and 10b of the tether remain within the user's body until after the excess tether has been cut, tissue anchors 50 and 56 and locks 60 and 66 can be slid onto the termini intracorporeally (e.g., transluminally).

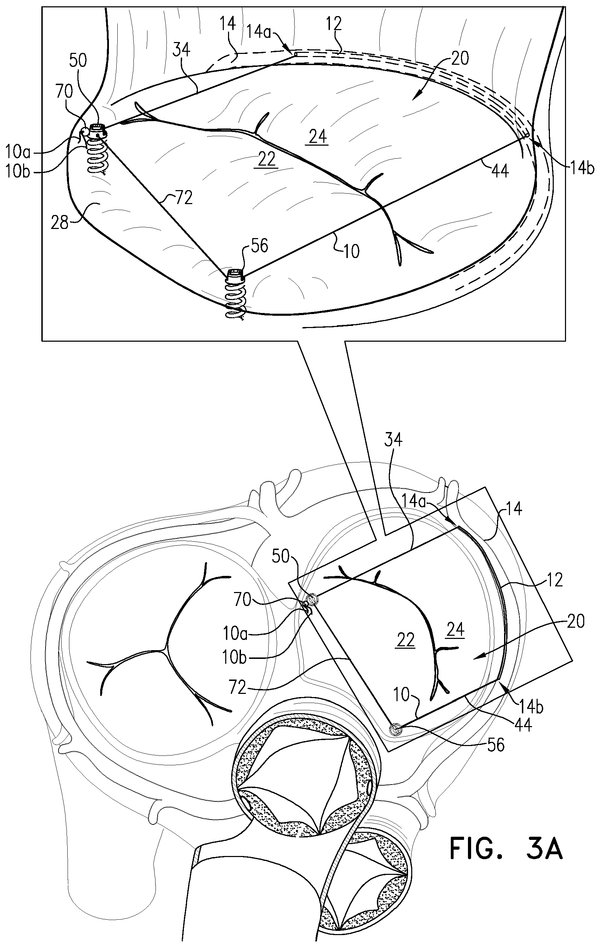

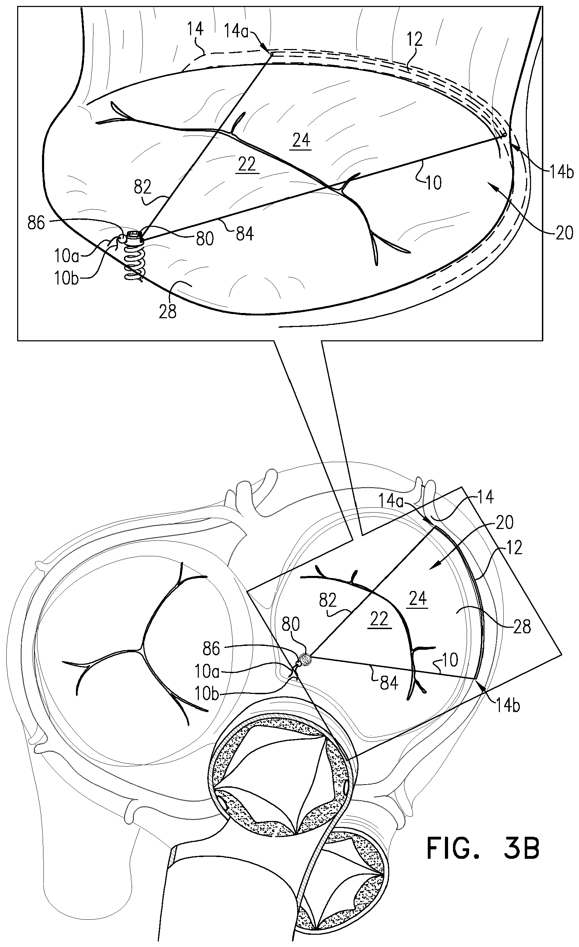

Figs. 3A and 3B are schematic sectional view illustrations of alternative implementations of the method of Figs. 2A to 2L, in accordance with some applications of the teachings herein. The method(s) can be performed on a living animal or on a simulation, such as on a cadaver, cadaver heart, simulator (e.g., with the body parts, heart, tissue, etc. being simulated), etc.

Fig. 3A demonstrates a method similar to that described hereinabove with respect to Figs. 2A to 2L. However, the example of Fig. 3A differs from the method previously described in the locking step of Fig. 2L. In the example of Fig. 3A, a single lock 70 is locked to both termini of the tether, e.g., in proximity to one of the tissue anchors, here shown as tissue anchor 50. In some applications, during or following tensioning of tether 10, second terminus 10b of the tether is drawn around or through second tissue anchor 56 to first tissue anchor 50, resulting in a segment 72 of the tether extending between the first and second tissue anchors. Lock 70 is then slid onto and along both termini of tether 10, to lock the termini adjacent first tissue anchor 50. As described hereinabove, following locking of the tension in the tether, excess tether can be cut and removed. In some applications, second terminus 10b may not be brought to anchor 50, but instead lock 70 may be slid over and along both termini to a location partway between anchors 50 and 56, such that the termini converge at the lock partway between the anchors, and the lock locks the tension in tether 10 by being locked to both termini in this position.

Fig. 3B demonstrates another method similar to that described hereinabove with respect to Figs. 2A to 2L. However, the example of Fig. 3B differs from the method previously described in the anchoring and locking steps of Figs. 2H-2L. In the example of Fig. 3B, a single tissue anchor replaces the first and second tissue anchors 50 and 56 of Figs. 2H to 2L. Specifically, a single tissue anchor 80 is slid over and along both termini of tether 10, and is anchored into tissue of the left atrium, for example into annulus 28, such that a first segment 82 and a second segment 84 of tether 10 extend from locations 14a and 14b, respectively, and across the atrium to converge at tissue anchor 80. Tissue anchor 80 can be driven by a driving tool, for example out of a catheter, substantially as described hereinabove with respect to driving tool 52.

Subsequently, the tension in tether 10 is modified substantially as described hereinabove, and the tension is locked using a lock 86 that is slid over and along the termini of the tether and is locked to the tether. As described hereinabove, following locking of the tension in the tether, the remainder of the tether can be snipped and removed.

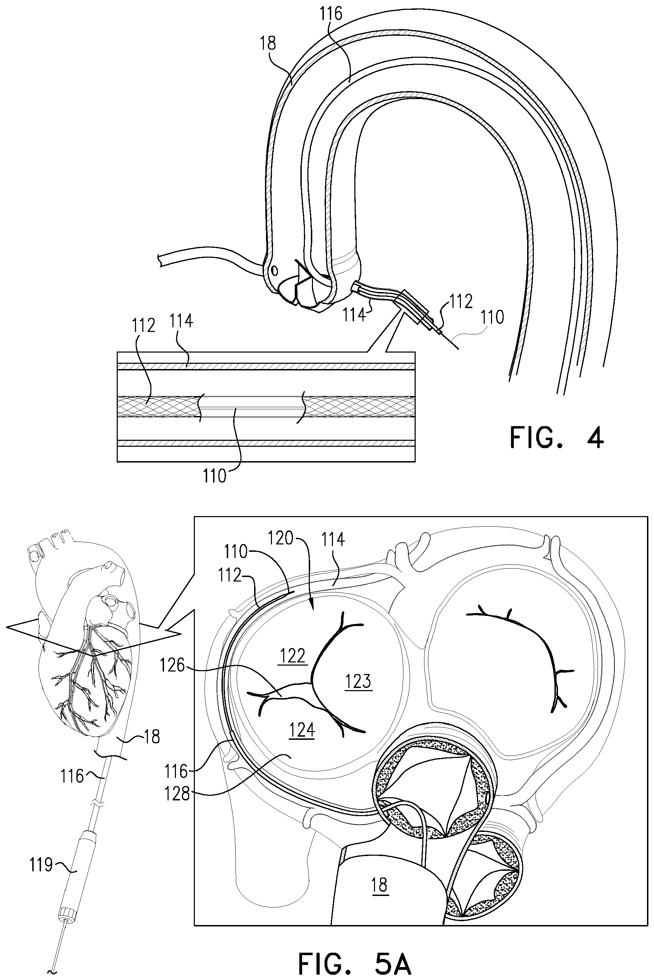

Fig. 4 is a schematic illustration of an example device according to an aspect of the teachings herein being delivered through the aorta of a subject into a coronary blood vessel, in this example a right coronary artery, of the heart of the subject, according to an example method. The method can be performed on a living animal or on a simulation, such as on a cadaver, cadaver heart, simulator (e.g., with the body parts, heart, tissue, etc. being simulated), etc.

As seen in Figure 4, a device according to the teachings herein includes a tether 110, which is surrounded by a support tube 112 and is slidable relative to the support tube. Tether 110 can be formed of any suitable material, such as metal, polymer, biomaterial wire, ribbon, or cord. Support tube 112 is typically flexible.

For treatment of the tricuspid valve (see reference numeral 120 in Figs. 5A-5L) of the subject, which is located between the right atrium and the right ventricle of the heart, tether 110 and support tube 112 are transluminally advanced into a right coronary artery 114 which at least partially circumscribes the tricuspid valve (see for example Fig. 5A). For some applications, and as shown in Fig. 4, a catheter 116 delivers tether 110 and support tube 112 via aorta 18 (e.g., transfemorally) and into right coronary artery 114.

In the illustrated example, a distal part of catheter 116 is advanced to the right coronary artery. Catheter 116 also has an extracorporeal proximal part 119 (e.g., as shown in Fig. 5A). The distal part of catheter 116 is guidable to the anatomical site, such as by being actively steerable (e.g., by being operatively coupled by one or more pullwires to proximal part 119, such as to a steering controller thereof), or by being passively guided and/or steered (e.g., by extending over or through another steerable element, such as an actively steerable catheter).

Reference is now made to Figs. 5A to 5L, which are schematic sectional view illustrations of steps of a method of treatment of tricuspid valve 120 of the heart of the subject using the device of Fig. 4, in accordance with some applications of the teachings herein. For example, the treatment of the tricuspid valve may be or include annuloplasty of the tricuspid valve. The method can be performed on a living animal or on a simulation, such as on a cadaver, cadaver heart, simulator (e.g., with the body parts, heart, tissue, etc. being simulated), etc.

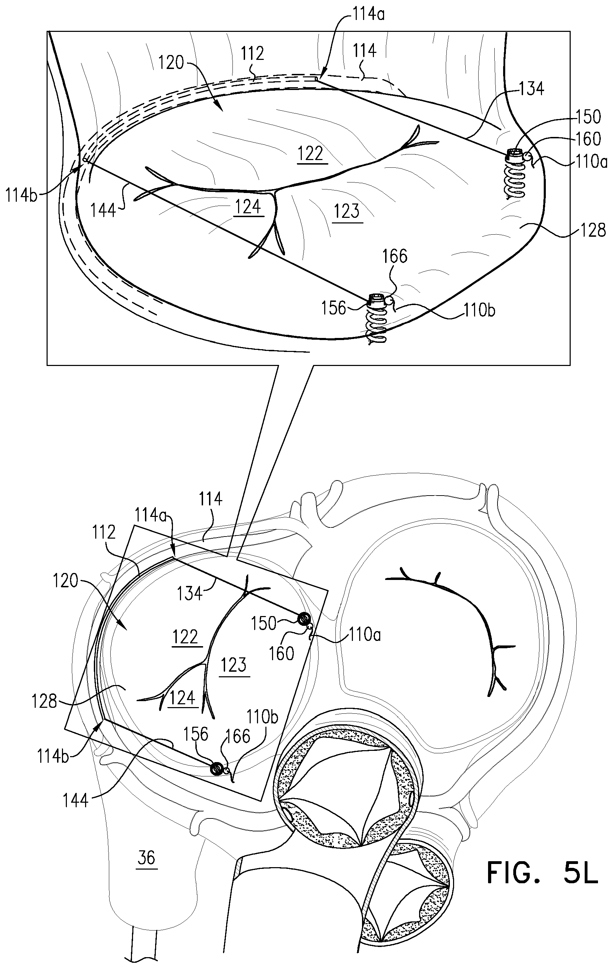

As seen in Fig. 5A, catheter 116 is used to advanced tether 110, and typically support tube 112, via aorta 18 into right coronary artery 114, as described hereinabove with respect to Fig. 4. As seen in the sectional portion of Fig. 5A, right coronary artery 114 at least partially circumscribes the tricuspid valve 120 of the subject's heart (e.g., is disposed alongside the annulus of the tricuspid valve). As seen, leaflets 122, 123, and 124 of the tricuspid valve 120 do not fully coapt, such that a gap 126 is formed therebetween. The treatment (e.g., annuloplasty) procedure described herein reshapes the tricuspid valve 120, such that the leaflets 122, 123, and 124 coapt and gap 126 is reduced or eliminated (see the end result in Fig. 5L). Tricuspid valve 120 includes an annulus 128, surrounding leaflets 122, 123, and 124.

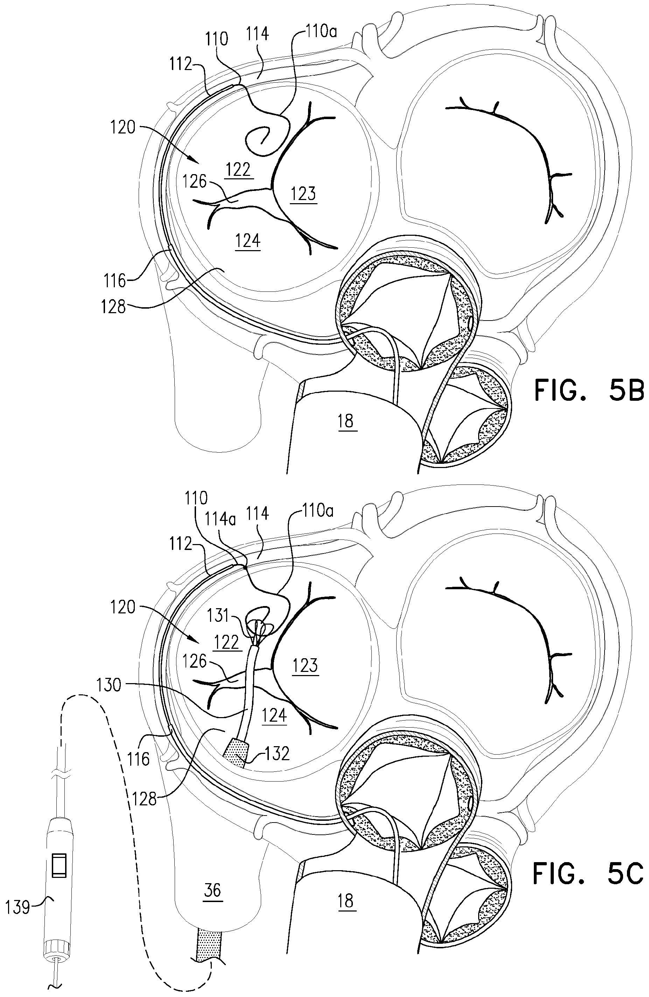

Turning to Fig. 5B, a hole is punctured (e.g., using a hollow needle) through the wall of the right coronary artery into the right atrium of the heart at a first location 114a, and a first terminus 110a of tether 110 is advanced into the atrium.

In some applications, tether 110 is advanced into right coronary artery 114 as shown in Fig. 5A, without support tube 112. In some applications, following advancement of first terminus 110a of tether 110 into the right atrium, support tube 112 can be advanced onto, and around, the tether.

As seen in Fig. 5C, a snare tool 130 terminating in a snare 131 is transluminally introduced into the right atrium of the heart and engages first terminus 10a of tether 110. In some applications, snare tool 130 is transluminally advanced into the right atrium using a second catheter 132. For example, snare tool 130 can be a component of second catheter 132 or can be a discrete device that is introduced via second catheter 132.

In some applications, and as shown, snare tool 130 is introduced into the right atrium through inferior vena cava 36 of the subject (e.g., transfemorally). In some applications, introduction of the snare tool can be via the superior vena cava.

In the illustrated example, a distal part of catheter 132 is advanced to the vena cava. Catheter 132 also has an extracorporeal proximal part 139 (e.g., as shown in Fig. 5C). The distal part of catheter 132 is guidable to the anatomical site, such as by being actively steerable (e.g., by being operatively coupled by one or more pullwires to proximal part 139, such as to a steering controller thereof), or by being passively guided and/or steered (e.g., by extending over or through another steerable element, such as an actively steerable catheter).

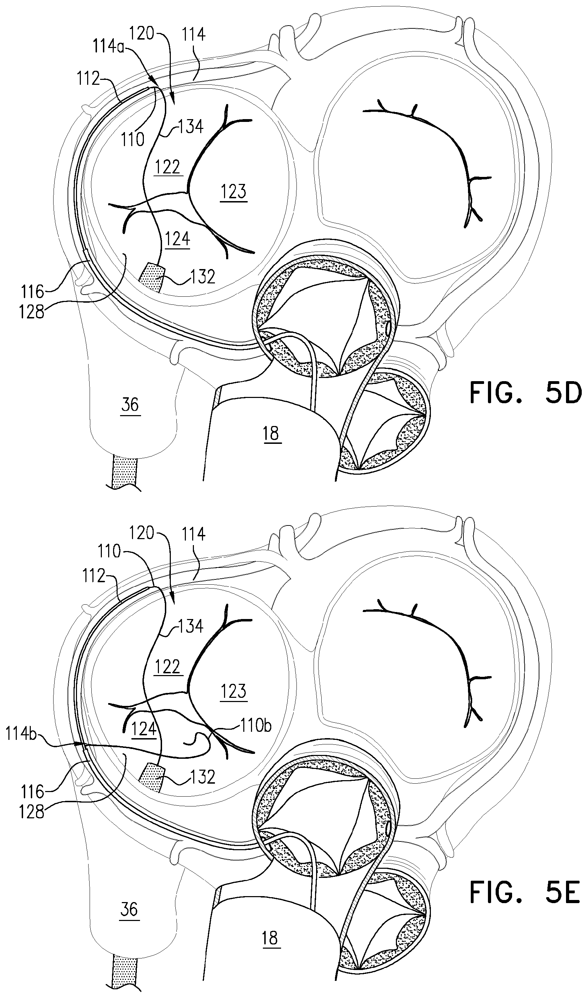

Snare 131 is then used to draw first terminus 110a of the tether out of the right atrium, such that a first segment 134 of the tether extends from puncture location 114a across the right atrium, as seen in Fig. 5D. In some applications, first terminus 110a is drawn through catheter 132, (e.g., by withdrawing snare tool 130 through the catheter) as shown in Fig. 5D.

In some applications, and as shown, snare 131 draws first terminus 110a from the right atrium through inferior vena cava 36 of the subject. In some applications, withdrawal can be via the superior vena cava.

As explained in further detail hereinbelow, in some applications of the step shown in Fig. 5D, first terminus 110a of tether 110 is drawn fully out of the subject's body. In some applications of the step shown in Fig. 5D, first terminus 110a is drawn out of the right atrium, but remains within the subject's body.

Turning to Fig. 5E, a second hole is punctured through the wall of the right coronary artery into the right atrium of the heart at a second location 114b, and a second terminus 110b of the tether is advanced into the atrium. As seen, support tube 112 can be disposed between first location 114a and second location 114b. In some applications, such as the example illustrated in Figs. 5A to 5L, first location 114a and second location 114b are selected to be near ends of gap 126 between leaflets 122, 123, and 124, or near ends of the coapting portion of the leaflets.

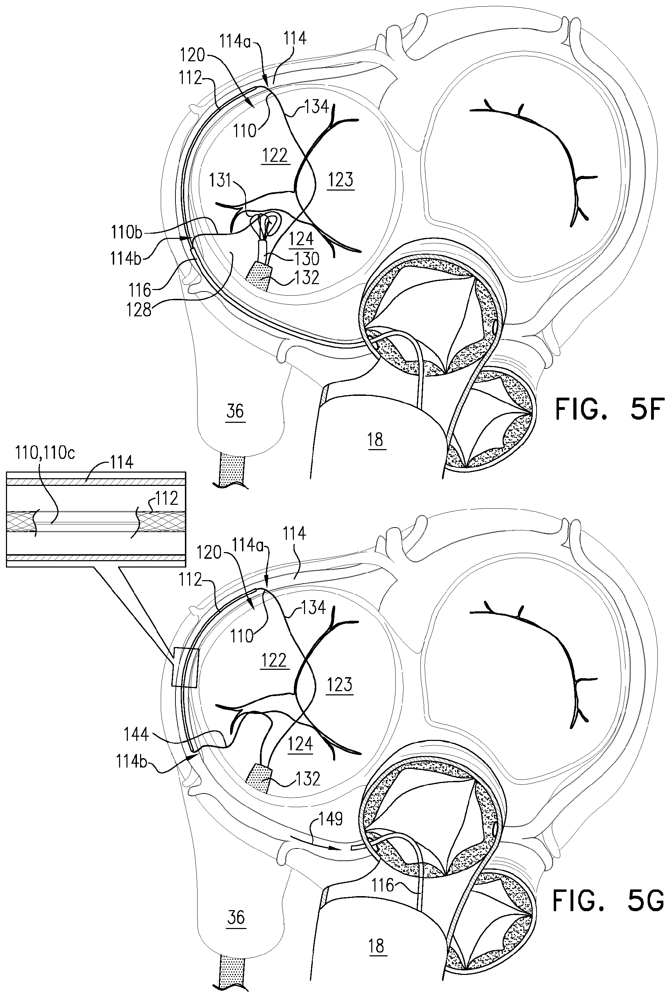

As seen in Fig. 5F, a snare tool, which can be snare tool 130 of Fig. 5C or can be a second snare tool, is transluminally introduced into the right atrium of the heart and engages second terminus 110b of the tether. The snare tool can be transluminally advanced into the right atrium out of second catheter 132 (e.g., as described with respect to Fig. 5C, mutatis mutandis), or out of another, third, catheter.

Snare 131 of snare tool 130 (or another snare of another snare tool) is then used to draw second terminus 110b of the tether out of the right atrium, such that a second segment 144 of the tether extends from second puncture location 114b across the right atrium, as seen in Fig. 5G.

In some applications, and as shown, snare 131 (or another snare) draws first terminus 110b from the right atrium through inferior vena cava 36 of the subject. In some applications, withdrawal can be via the superior vena cava.

In some applications, for example when the same snare and catheter are used for drawing both the first and second termini of tether 110, the first and second termini are both drawn from the right atrium via the same route, i.e., via the same one of the superior and inferior vena cava. As shown in Fig. 5G, for such examples, tether 110 forms a loop from catheter 132, across the right atrium, into and along right coronary artery 114, back through the atrium, and into the catheter. This loop includes (i) segment 134 extending from catheter 132 across the atrium to location 114a, (ii) a bight 110c of tether 110 extending between location 114a and location 114b (e.g., within support tube 112), and (iii) segment 144 extending from location 114b back through the atrium and into the catheter.

In some applications, for example when snare 131 is used to draw first terminus 110a through catheter 132, and a separate snare is used to draw second terminus 110b through a separate catheter, each of the termini of tether 110 can be drawn out of the right atrium using a different route. For example, first terminus 110a may be drawn through the superior vena cava, while second terminus 110b may be drawn through the inferior vena cava.

As explained in further detail hereinbelow, in some applications of the step shown in Fig. 5G, second terminus 110b of tether 110 is drawn fully out of the subject's body. In some applications of the step shown in Fig. 5G, second terminus 110b is drawn out of the right atrium, but remains within the subject's body. In some applications, the first and second termini of tether 110 are drawn out of the right atrium to the same extent (i.e., both are pulled fully out of the subject's body, or both are pulled out of the right atrium but remain in the subject's body).

In some applications, once both termini of tether 110 are drawn from the right atrium, catheter 116 can be removed from right coronary artery 114, for example by retraction of the catheter through aorta 18.

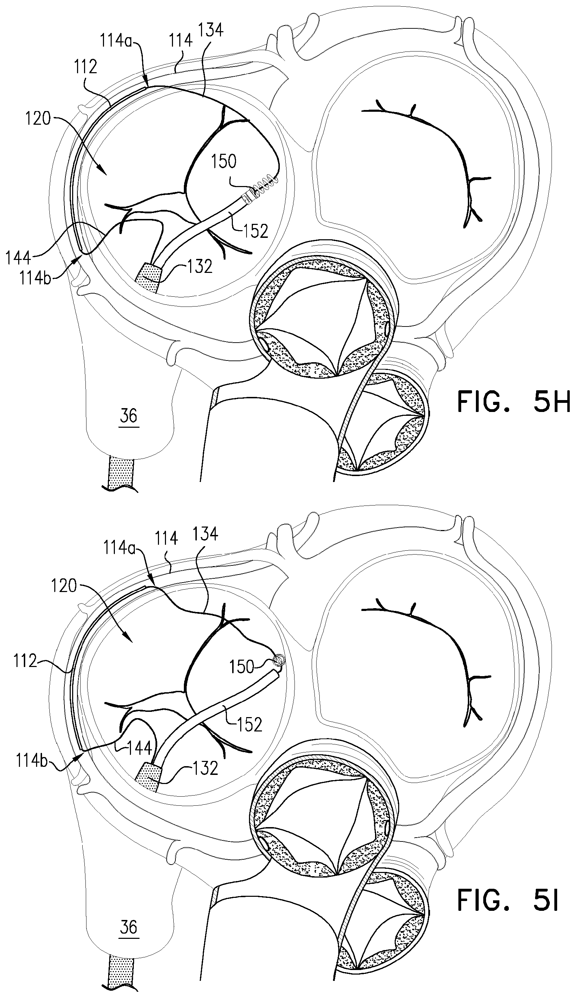

Turning to Fig. 5H, a first tissue anchor 150, e.g., driven by a driving tool 152, is slid over and along first terminus 110a toward first segment 134. First tissue anchor 150 is anchored into tissue of the right atrium, for example into annulus 128, such that first segment 134 now extends between right coronary artery 114 and first tissue anchor 150, as seen in Fig. 5I. First tissue anchor 150 can be anchored across tricuspid valve 120 from right coronary artery 114 (e.g., from support tube 112), such as in proximity to the root of septal leaflet 123 or a commissure at which the septal leaflet meets posterior leaflet 122 or anterior leaflet 124. At this stage, first segment 134 may be slack.

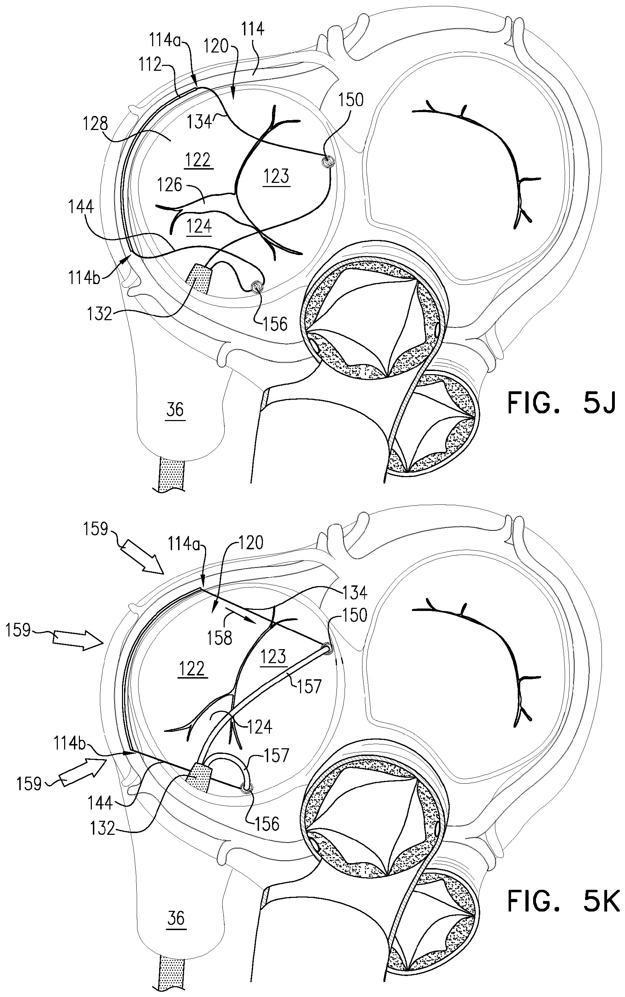

As seen in Fig. 5J, a second tissue anchor 156 is slid over and along second terminus 110b toward second segment 144. Second tissue anchor 156 is anchored into tissue of the right atrium, for example into annulus 128, such that second segment 144 now extends between right coronary artery 114 and the second tissue anchor. Second tissue anchor 156 can be anchored across tricuspid valve 120 from right coronary artery 114 (e.g., from support tube 112), such as in proximity to the root of septal leaflet 123 or a commissure at which the septal leaflet meets posterior leaflet 122 or anterior leaflet 124. At this stage, second segment 144 may be slack. Second tissue anchor 156 can be driven by driving tool 152, or by another anchor driving tool.

Each of anchors 150 and 156 can have a head that is slidably couplable to tether 110, e.g., by the head comprising an eyelet that is threadable onto the tether. Each of anchors 150 and 156 can have a tissue-engaging element, which can be a helical screw-in tissue-engaging element (as described herein) or can be another type of tissue-engaging element, such as a dart or a staple. For some applications, each of anchors 150 and 156 can comprise an anchor described in one or more of the following:
- US Patent Application 17/145,258 to Kasher et al., filed January 8, 2021, which published as US 2021/0145584;
- US Provisional Patent Application 63/147,699 to Shafigh et al., filed February 9, 2021; and
- International Patent Application PCT/IB2022/051099 to Shafigh et al., filed February 8, 2022.

Driving tool 152 can be advanced via second catheter 132 (e.g., as shown in Fig. 5H), or from another catheter. In some applications, and as shown, driving tool 152 is rotatable, and anchors the tissue anchor(s) by screwing the tissue anchor(s) into the tissue of annulus 128. Following anchoring of tissue anchors 150 and 156, driving tool 152 (and, if one is used, another driving tool) can be retracted from the right atrium, for example via second catheter 132.

Turning to Fig. 5K, the tension in tether 110 is modified (e.g., tension is applied to the tether). In some applications, this is done by pulling on one or both of termini 110a and 110b. This pulling is indicated by arrows 158. Tensioning can be facilitated by one or more transluminally introduced tools 157 that can, for example, provide an opposing force against anchors 150 and 156. The application of tension to tether 110 can pull support tube 112 against the wall of right coronary artery 114 that is closest to the atrium.

Modification of the tension in tether 110 causes reshaping of the tricuspid valve 120, for example in the direction of arrows 159. As shown in Fig. 5L, the reshaping of the tricuspid valve improves coaptation between leaflets 122, 123, and 124 of the valve, thereby reducing (e.g., closing) gap 126.

In some applications, support tube 112 is designed and configured to distribute force, applied by tether 110, across the wall of right coronary artery 114, so as to prevent the tether from damaging the wall of the right coronary artery and/or the wall of the atrium. In some applications, support tube 112 maintains puncture locations 114a and 114b at a fixed distance from one another, for example by preventing tether 110 from cutting (e.g., in a similar manner to a cheese wire) through the wall of the right coronary artery from one of the locations toward the other.

Following modification to the tension of the tether and reshaping of the valve, the tension in the tether is locked. In the example illustrated in Fig. 5L, a first lock 160 (which can optionally be referred to as a stopper) is slid along first terminus 110a of tether 110, and is locked to the tether, typically in proximity to first anchor 150. A second lock 166 is slid along second terminus 110b of tether 110, and is locked to the tether, typically in proximity to second anchor 156. Locks 160 and 166 can lock the tension in tether 110 by inhibiting the tether (e.g., termini 110a and 110b) from sliding past anchors 150 and 156, e.g., by the locks abutting the anchors.

In some applications, excess tether is then cut and removed from the right atrium, for example by retraction of catheter 132. The advancement of locks 160 and 166, and cutting of excess tether, can be performed using tools 157.

In some applications, the modification of the tension in tether 110 can be accomplished by pulling on both termini 110a and 110b.

In some applications, the modification of the tension in tether 110 can be accomplished by initially locking one of termini 110a and 110b adjacent the respective one of tissue anchors 150 and 156, and subsequently pulling on the other one of termini 110a and 110b to modify the tension in the tether. Once the desired tension of the tether is achieved, the other of termini 110a and 110b is locked adjacent the respective one of tissue anchors 150 and 156. For example, first terminus 110a can be locked adjacent tissue anchor 150, following which the end user or physician cab pull on terminus 110b to change the tension in the tether, and then locks second terminus 110b adjacent second tissue anchor 156.

In applications in which the termini 110a and 110b of the tether are drawn out of the user's body, as described hereinabove, the tissue anchors 150 and 156, as well as locks 160 and 166, can be slid onto the termini of the tether extracorporeally. In some applications, in which the termini 110a and 110b of the tether remain within the user's body until after the excess tether has been cut, tissue anchors 150 and 156 and locks 160 and 166 can be slid onto the termini intracorporeally (e.g., transluminally).

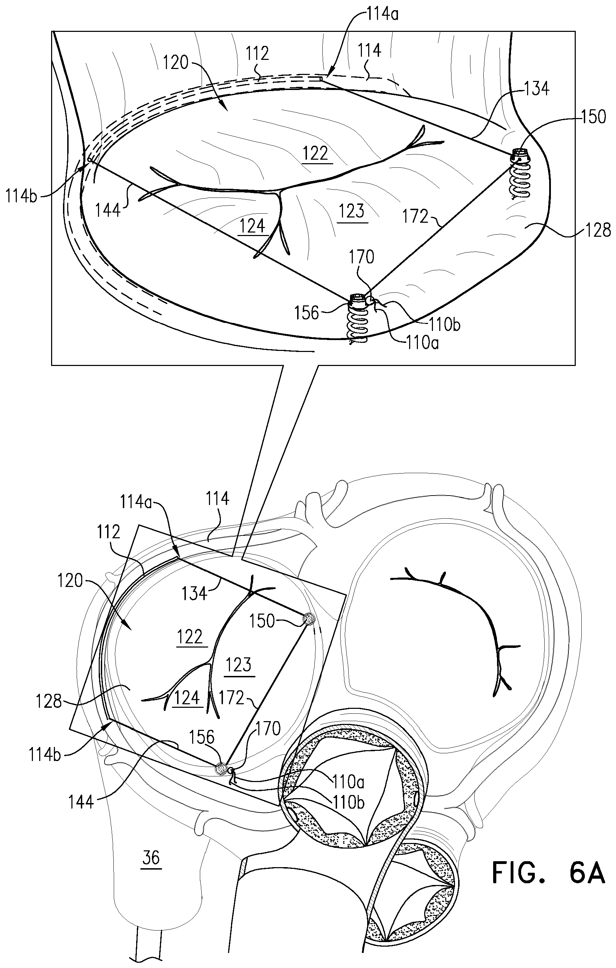

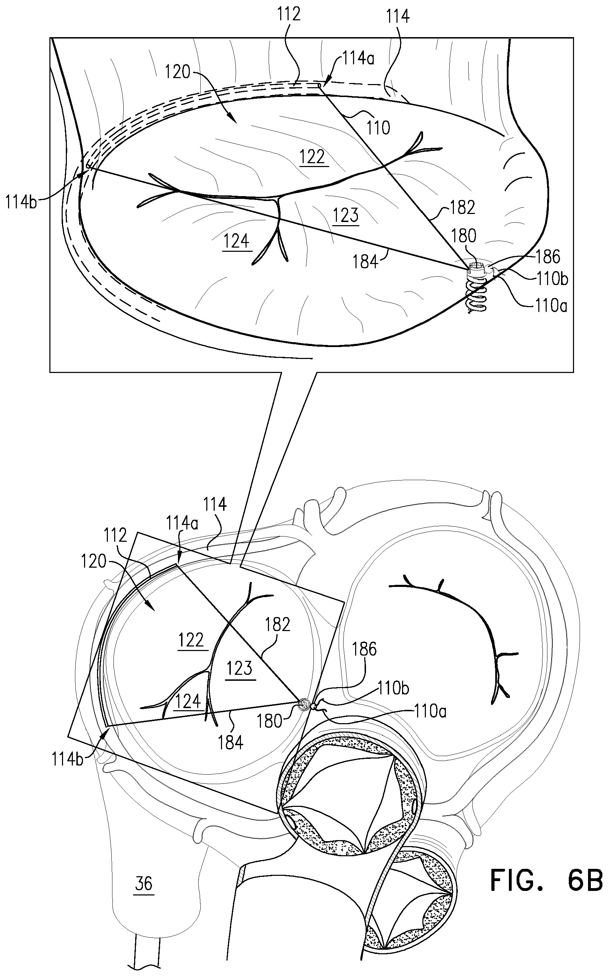

Figs. 6A and 6B are schematic sectional view illustrations of alternative implementations of the method of Figs. 5A to 5L, in accordance with some applications of the teachings herein. The method(s) can be performed on a living animal or on a simulation, such as on a cadaver, cadaver heart, simulator (e.g., with the body parts, heart, tissue, etc. being simulated), etc.

Fig. 6A demonstrates a method similar to that described hereinabove with respect to Figs. 5A to 5L. However, the example of Fig. 6A differs from the method previously described in the locking step of Fig. 5L. In the example of Fig. 6A, a single lock 170 is locked to both termini of the tether, e.g., in proximity to one of the tissue anchors, here shown as tissue anchor 150. In some applications, during or following tensioning of tether 110, first terminus 110a of the tether is drawn around or through first tissue anchor 150 to second tissue anchor 156, resulting in a segment 172 of the tether extending between the first and second tissue anchors. Lock 170 is then slid onto and along both termini of tether 110, to lock the termini adjacent second tissue anchor 156. As described hereinabove, following locking of the tension in the tether, excess tether can be cut and removed. In some applications, second terminus 110b may not be brought to anchor 150, but instead lock 170 can be slid over and along both termini to a location partway between anchors 150 and 156, such that the termini converge at the lock partway between the anchors, and the lock locks the tension in tether 110 by being locked to both termini in this position.

Fig. 6B demonstrates another method similar to that described hereinabove with respect to Figs. 5A to 5L. However, the example of Fig. 6B differs from the method previously described in the anchoring and locking steps of Figs. 5H-5L. In the example of Fig. 6B, a single tissue anchor replaces the first and second tissue anchors 150 and 156 of Figs. 5H to 5L. Specifically, a single tissue anchor 180 is slid over and along both termini of tether 110, and is anchored into tissue of the right atrium, for example into annulus 128, such that segments 182 and 184 extend from locations 114a and 114b and across the atrium to converge at tissue anchor 180. Tissue anchor 180 can be driven by a driving tool, for example out of a catheter, substantially as described hereinabove with respect to driving tool 152.

Subsequently, the tension in tether 110 is modified substantially as described hereinabove, the tension is locked using a lock 186 that is slid over and along the termini of the tether and is locked to the tether. As described hereinabove, following locking of the tension in the tether, excess tether can be cut and removed.

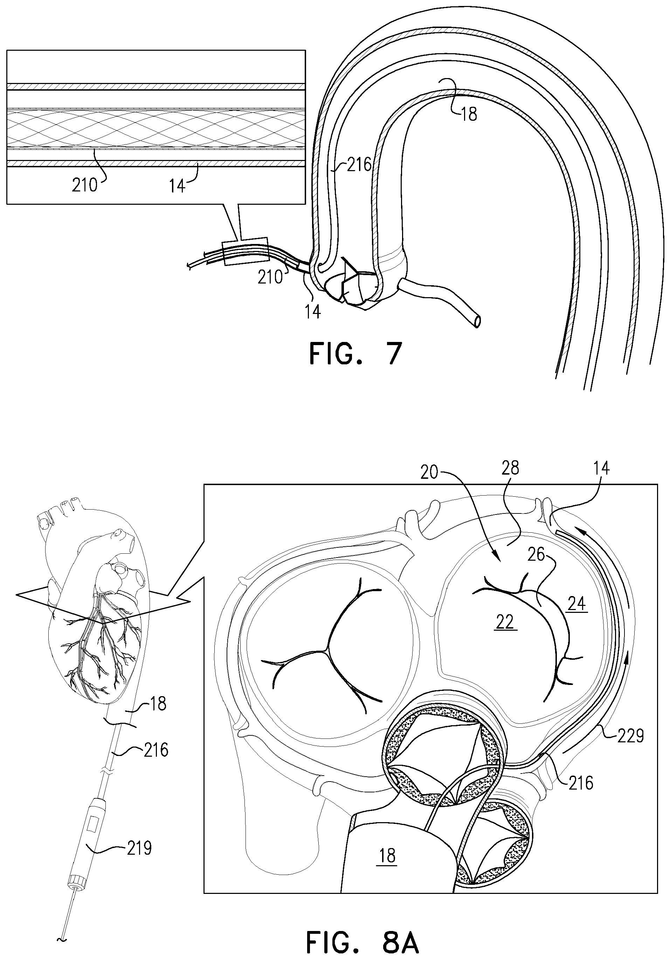

Reference is now made to Figs. 7 and 8A-K, which are schematic illustrations of a device, and techniques for use therewith, for protecting a coronary blood vessel or artery during treatment of an atrioventricular heart valve of a subject, according to some applications of the teachings herein.

As seen in Figure 7, a device according to the teachings herein includes an elongate tube 210 comprising (e.g., formed of) a radiopaque material. In some applications, elongate tube 210 is flexible. In some applications, such as that shown, elongate tube 210 can include, or be formed of, a metal frame or a mesh frame. For treatment of the mitral valve (see reference numeral 20 in Figs. 8A-8K) of the subject, which is located between the left atrium and the left ventricle of the heart, elongate tube 210 is transluminally advanced into a coronary blood vessel, for example, a left coronary artery 14. For some applications, and as shown in Fig. 7, a catheter 216 delivers the elongate tube 210 via aorta 18 (e.g., transfemorally) and into left coronary artery 14.

In the illustrated example, a distal part of catheter 216 is advanced to the left coronary artery. Catheter 216 also has an extracorporeal proximal part 219 that can comprise a handle (e.g., as shown in Fig. 8A). The distal part of catheter 216 is guidable to the left coronary artery, such as by being actively steerable (e.g., by being operatively coupled by one or more pullwires to proximal part 219, such as to a steering controller thereof), or by being passively guided and/or steered (e.g., by extending over or through another steerable element, such as an actively steerable catheter).

Figs. 8A to 8K are schematic sectional view illustrations of steps of a method of preventing damage to the left coronary artery during treatment of mitral valve 20 of the heart using the device of Fig. 7, in accordance with some applications of the teachings herein. For example, the treatment of the mitral valve may be or include annuloplasty of the mitral valve. The method can be performed on a living animal or on a simulation, such as on a cadaver, cadaver heart, simulator (e.g., with the body parts, heart, tissue, etc. being simulated), etc.

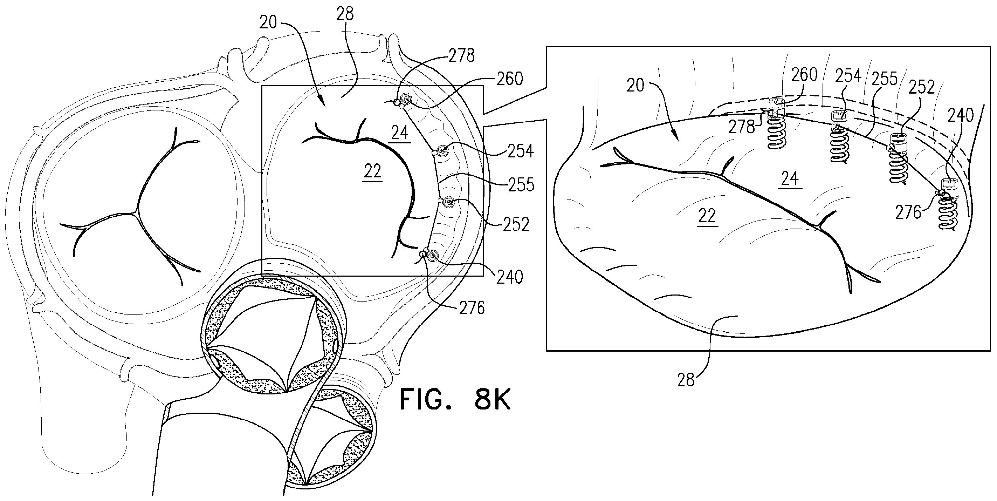

As seen in the sectional portion of Fig. 8A, left coronary artery 14 at least partially circumscribes the mitral valve 20 of the subject's heart. As seen, leaflets 22 and 24 of the mitral valve do not fully coapt, such that a gap 26 is formed therebetween. The treatment (e.g., annuloplasty) procedure described herein, reshapes the mitral valve 20, such that the leaflets 22 and 24 coapt and gap 26 is reduced or eliminated (see the end result in Fig. 8K). Mitral valve 20 includes an annulus 28, surrounding leaflets 22 and 24.

The following description relates to a transluminal annuloplasty procedure, in which a tether is secured in an arc around the valve being treated by anchoring multiple tissue anchors to the tissue of the annulus in a series around the valve. The annulus is then contracted by tensioning the tether, to which the anchors are slidably coupled. Examples of such annuloplasty methods are described in US Patent Application 14/437,373 to Sheps et al., filed April 21, 2015, which published as US 2015/0272734; US Patent Application 15/782,687 to Iflah et al., filed October 12, 2017, which published as US 2018/0049875; US Patent Application 16/534,875 to Brauon et al., filed August 7, 2019, which published as US 2020/0015971; and US Patent Application 17/145,258 to Kasher et al., filed January 8, 2021, which published as US 2021/0145584. However, elongate tube 210 of the disclosed technology can be used, substantially as described, also when implementing other techniques that include anchoring a tissue anchor within the atrium. These method can be performed on a living animal or on a simulation, such as on a cadaver, cadaver heart, simulator (e.g., with the body parts, heart, tissue, etc. being simulated), etc.

As seen in Fig. 8A, catheter 216 is used to advanced elongate tube 210 into left coronary artery 14, in the direction of arrows 229, as described hereinabove with respect to Fig. 7. In some applications, elongate tube 210 can be advanced transluminally over a guide wire.

Elongate tube 210 is advanced to the left coronary artery while the elongate tube is compressed, for example by being enclosed within an external sheath, here shown as the body of catheter 216.

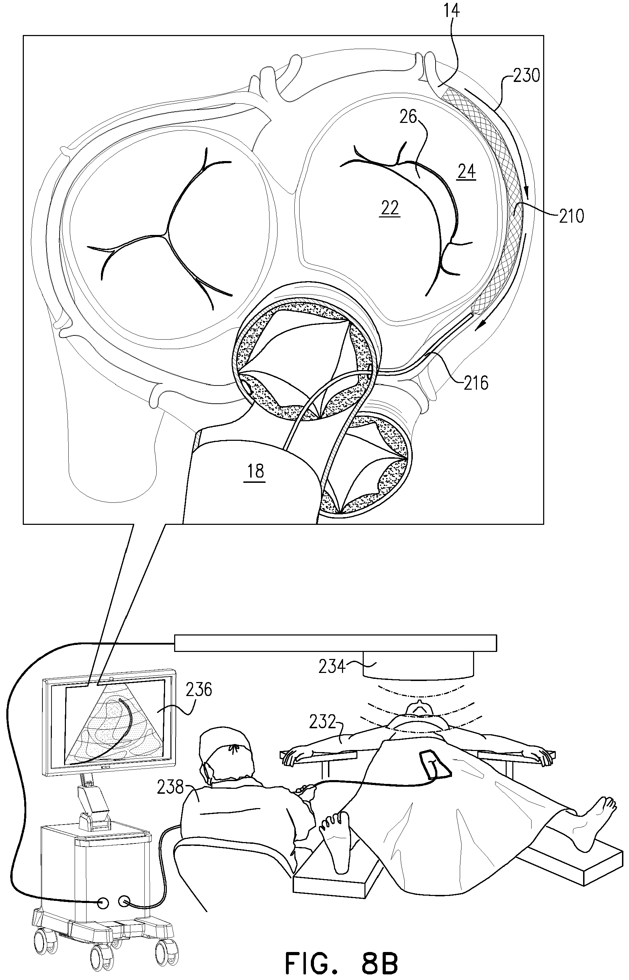

Turning to Fig. 8B, the external sheath, or catheter 216, can be retracted in a proximal direction, indicated by arrows 230, allowing elongate tube 210 to expand radially.

In some applications, radial expansion of flexible tube 210 causes the tube to become longitudinally contracted. For example, flexible tube 210 can be self-expanding. In some applications, the flexible tube 210 comprises a self-expanding structure, such as a stent, stent-like structure, braided structure, woven structure, balloon, etc. Alternatively or additionally, flexible tube 210 can be expanded by longitudinally contracting the tube, e.g., by pulling one end of the flexible tube toward to the other end. For example, the distal end of the flexible tube can be pulled while applying a reference force to the distal end of the flexible tube, thereby to radially expand the compressed structure (while longitudinally contracting the tube). In some applications, a pulling element or tool is transluminally advanced into left coronary artery 14 in order to pull one end of flexible tube 210 relative to the opposing end.

In some applications, flexible tube 210 is stent-like. In some applications, flexible tube 210 has a cellular structure, e.g., cut from a tube. In some applications, flexible tube 210 has a braided structure.

In some applications, the outer diameter of elongate tube 210, when expanded, is substantially similar to the inner diameter of left coronary artery 14, e.g., such that the elongate tube is in circumferential contact with the inner surface of the left coronary artery. In some applications, elongate tube 210 is sufficiently long to extend at least a quarter of the way around annulus 28 of mitral valve 20.

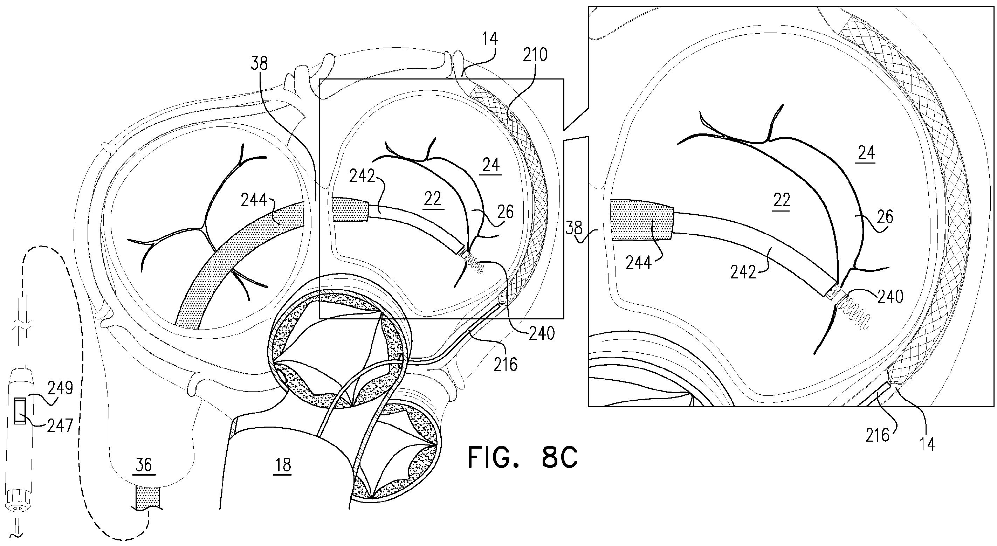

The introduction of elongate tube 210 into the left coronary artery as shown in Figs. 7 and 8A, the expansion thereof shown in Fig. 8B, and the insertion of tissue anchors as described hereinbelow with respect to Figs. 8C to 8K, are performed on the subject 232 transluminally, typically facilitated by imaging (e.g., fluoroscopy). For example, an imaging device 234 can deliver real time images of the procedure to a display screen 236, visible to an end user or physician 238 who can also be controlling catheter 216, and other catheters introduced at other stages of the treatment. Elongate tube 210, being radiopaque, is visible to end user or physician 238 in the images provided on display screen 236.

As seen in Fig. 8C, a first tissue anchor 240, e.g., driven by a driving tool 242 extending distally out of a second catheter 244, is advanced to the left atrium of the heart of the subject, the left atrium being upstream of mitral valve 20. Second catheter 244 can advance the driving tool 242 to the left atrium via an inferior vena cava 36 and then via an opening in the interspatial septum 38. In some applications, advancement of the driving tool can be via the superior vena cava.

In the illustrated example, a distal part of catheter 244 is advanced to the vena cava. Catheter 244 also has an extracorporeal proximal part 249, which can include a handle (e.g., as shown in Fig. 8C). The distal part of catheter 244 is guidable to the anatomical site, such as by being actively steerable (e.g., by being operatively coupled by one or more pullwires to proximal part 249, such as to a steering controller thereof), or by being passively guided and/or steered (e.g., by extending over or through another steerable element, such as an actively steerable catheter).

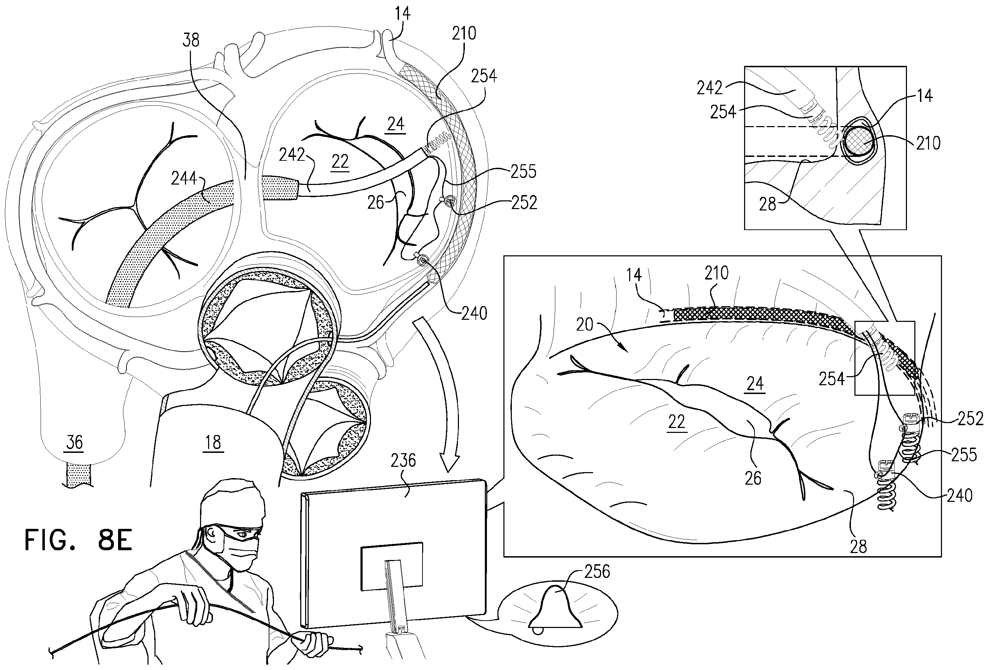

Tissue anchor 240 can have a head that is slidably coupled to a tether 255 (Fig. 8E), e.g., by the head comprising an eyelet that is threadable onto the tether. Tissue anchor 240 can have a tissue-engaging element, which can be a helical screw-in tissue-engaging element (as shown) or can be another type of tissue-engaging element, such as a dart or a staple. For some applications, tissue anchor 240 can comprise an anchor described in one or more of the following:
- US Patent Application 17/145,258 to Kasher et al., filed January 8, 2021, which published as US 2021/0145584;
- US Provisional Patent Application 63/147,699 to Shafigh et al., filed February 9, 2021; and
- International Patent Application PCT/IB2022/051099 to Shafigh et al., filed February 8, 2022.

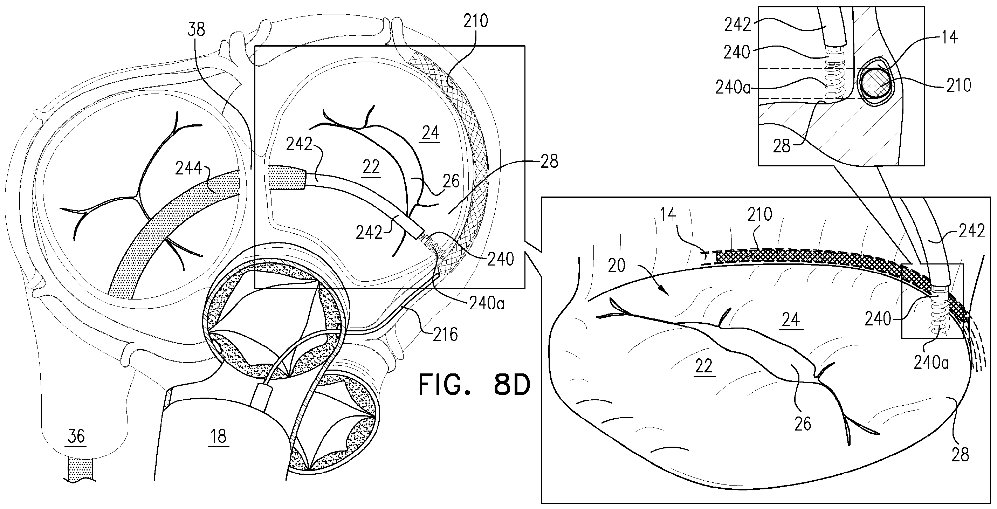

Turning to Fig. 8D, it is seen that driving tool 242 anchors first tissue anchor 240 into tissue of annulus 28 of mitral valve 20. In some applications, driving tool 242 is rotatable, such that rotation of the driving tool screws in a tissue-engaging portion of 240a of first tissue anchor 240 into the tissue of annulus 28.

For applications in which the introduction and anchoring of first tissue anchor 240 is facilitated by fluoroscopic imaging of the heart, as described above, because elongate tube 210 is radiopaque and typically substantially fills the diameter of left coronary artery 14, end user or physician 238 can identify the location and bounds of the left coronary artery based on the location of the elongate tube, visible on display 236. Thus, the end user or physician can avoid tissue anchor 240 contacting or piercing the left coronary artery. In some applications, during anchoring of first tissue anchor 240, and of additional tissue anchors as described hereinbelow, the body of the subject is devoid of fluid contrast agents, e.g., the procedure can be performed without the introduction of a fluid contrast agent into the subject.

Turning to Fig. 8E, a second tissue anchor 252 has been anchored into the tissue of annulus 28, and a third tissue anchor 254 is about to be anchored. Tissue anchors 252 and 254 are similar in structure to tissue anchor 240 described hereinabove, and are slidably coupled to tether 255, which extends distally out of driving tool 242 or out of second catheter 244. The placement of anchors 240, 252, and 254 is facilitated by fluoroscopic imaging of the heart of the subject, so that the radiopaque elongate tube 210 is visible to the operating physician or end user, and thus the physician or end user knows the location and bounds of left coronary artery 14 and can avoid damage thereto.

In some applications, elongate tube 210 is formed of an electrically conductive material, and driving tool 242 is electrically coupled to a control subsystem 247, (for example disposed in or connected to proximal part 249). In some applications, contact between a tissue anchor, such as tissue anchor 254, and elongate tube 210, is detected by control subsystem 247, which provides a signal (e.g., on or via display 236) such as an audible or visual signal, indicated in Fig. 8E by reference numeral 256. In such applications, the end user or physician 238 can monitor for signal 256, indicating proximity or contact between a tissue anchor and elongate tube 210. In response to identification of detectable signal 256, the end user or operating physician may reposition the tissue anchor, here shown as third tissue anchor 254, in order to avoid damaging left coronary artery 14, and drives the third tissue anchor into the tissue of annulus 28 at a modified location.

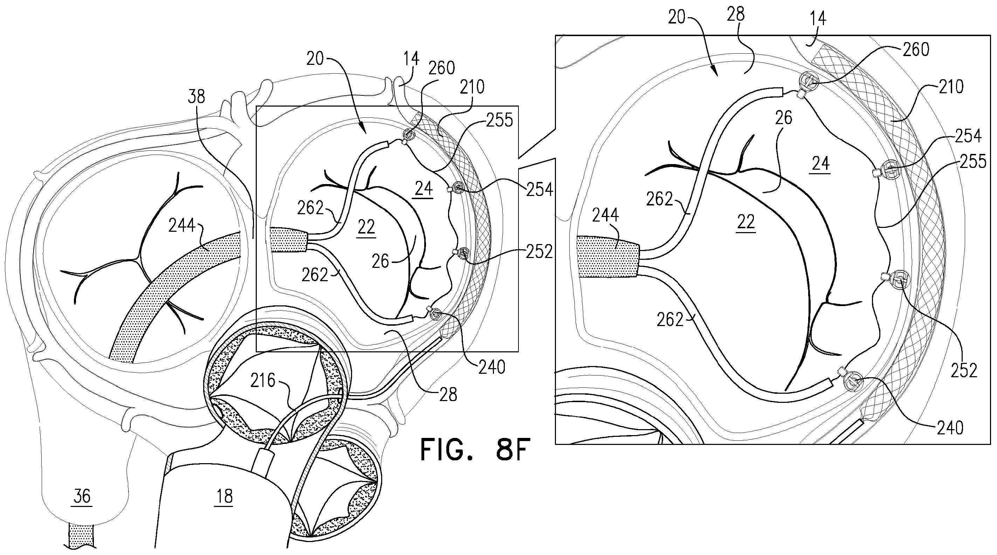

Fig. 8F shows mitral valve 20 following anchoring of tissue anchors 240, 252, and 254 shown previously, and of a fourth, additional tissue anchor 260, similar in structure to tissue anchor 240 and slidably coupled to tether 255. As seen in Fig. 8F, a pair of tools 262 extend out of catheter 244 along both ends of tether 255. As is explained hereinbelow, tools 262 will be used to modify the tension in tether 255, lock the tension in the tether, and cut the tether.

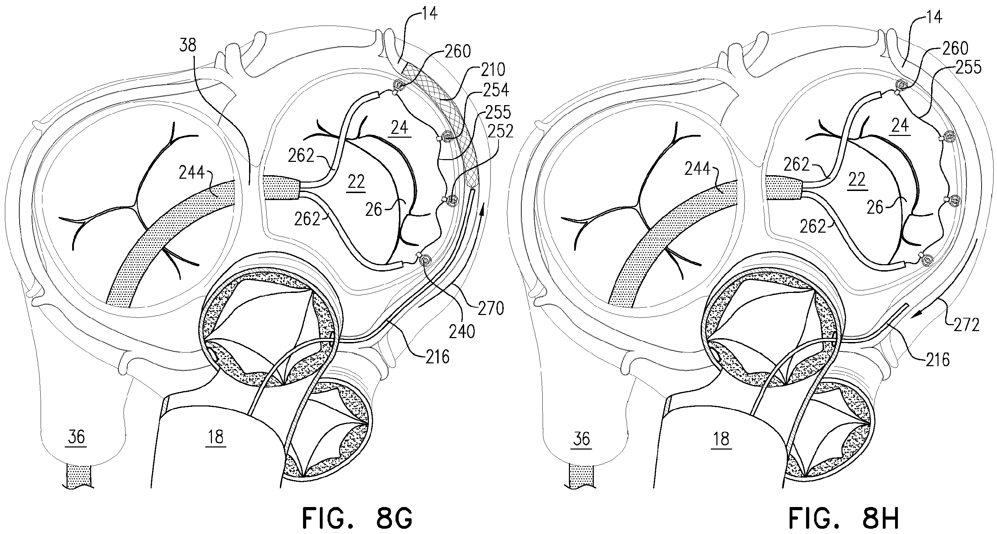

Following installation of all the required tissue anchors, elongate tube 210 can be removed from the left coronary artery. An exemplary method for removing elongate tube 210 is shown in Figs. 8G and 8H.

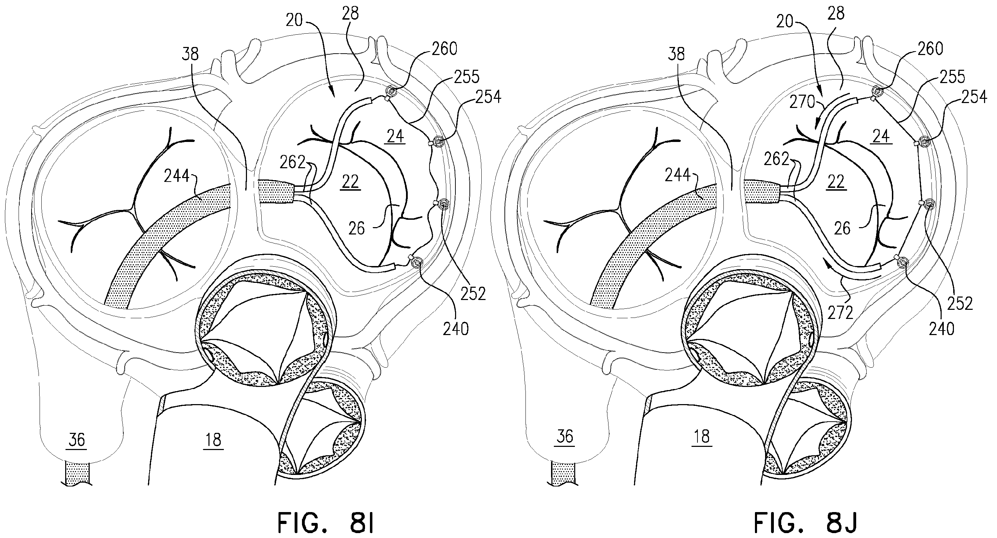

In Fig. 8G, the external sheath, or catheter 216, is advanced distally over elongate tube 210, in the direction of arrow 270, causing the elongate tube to compress within the sheath. Other mechanisms of compression of elongate tube 210 can also be employed. Following compression of elongate tube 210, catheter 216 is retracted from the left coronary artery in a direction indicated by arrow 272, as seen in Fig. 8H, and is removed from the body of the subject via the aorta 18. Fig. 8I shows the mitral valve of the subject's heart following removal of catheter 216, and prior to tensioning of tether 255, as explained hereinbelow.

Turning to Fig. 8J, the tension in tether 255 is modified (e.g., tension is applied to the tether). In some applications, this is done by pulling ends of the tether. This pulling is indicated by arrows 274. Tensioning can be facilitated by one or more transluminally introduced tools 262 that can, for example, provide an opposing force against anchors 240 and 260. Modification of the tension in tether 255 causes reshaping of mitral valve 20. As shown in Fig. 8K, the reshaping of the mitral valve causes leaflets 22 and 24 of the valve to fully coapt, and gap 26, which is visible in Figs. 8A-8I, is closed.

Following modification to the tension of the tether and reshaping of the valve, the tension in the tether is locked. In the example illustrated in Fig. 8I, a first lock 276 (which can optionally be referred to as a stopper), is slid along one end of tether 255, and is locked to the tether, typically in proximity to tissue anchor 240. A second lock 278 is slid along an opposing end of tether 255, and is locked to the tether, typically in proximity to tissue anchor 260. Locks 276 and 278 can lock the tension in tether 255 by inhibiting the tether (e.g., termini 10a and 10b) from sliding past anchors 240 and 260, e.g., by the locks abutting the anchors.

In some applications, excess tether is then cut and removed from the left atrium, for example by retraction of catheter 244 via the septum 38 and vena cava 36.

In some applications, the modification of the tension in tether 255 can be accomplished by pulling on both ends of the tether.

In some applications, the modification of the tension in tether 255 can be accomplished by initially locking one of the ends of the tether adjacent the respective one of tissue anchors 240 and 260, and subsequently pulling on the other end of tether 255 to modify the tension in the tether. Once the desired tension of the tether is achieved, the other end of the tether is locked adjacent the respective one of tissue anchors 240 and 260. For example, the first end can be locked adjacent tissue anchor 240, following which the end user or physician can pull on the second end to change the tension in the tether, and then locks the second end adjacent tissue anchor 260.

In some applications, the tether can be advanced into the body together with one lock (e.g., one lock is pushed into the body along with the tether, possibly already locked to the tether). In some cases, the first anchor can be advanced together with the first lock attached to the tether adjacent the first anchor, such that the end user or physician only interacts with the second end of the tether, and with the second lock.

It is appreciated that while the description of Figs. 7 and 8A-8K herein is provided with respect to treatment of the mitral valve (e.g., annuloplasty), the method and device of the teachings herein can be used, mutatis mutandis, during treatment of the tricuspid valve, by advancing flexible tube 210 into coronary blood vessel, for example, a right coronary artery, and carrying out treatment of the tricuspid valve via the vena cava, for example as described hereinabove with respect to Figs. 4 and 5A-5L. In some applications, the flexible tube can be advanced into the right coronary artery via a coronary ostia at the aortic root of aorta 18 of the subject. In some applications, elongate tube 210 is sufficiently long to extend around at least a majority of the annulus of the tricuspid valve (see for example Figs. 4-5L), and the anchoring of tissue anchors 240, 252, 254, and 260, or any other number of tissue anchors, is into the annulus of the tricuspid valve.

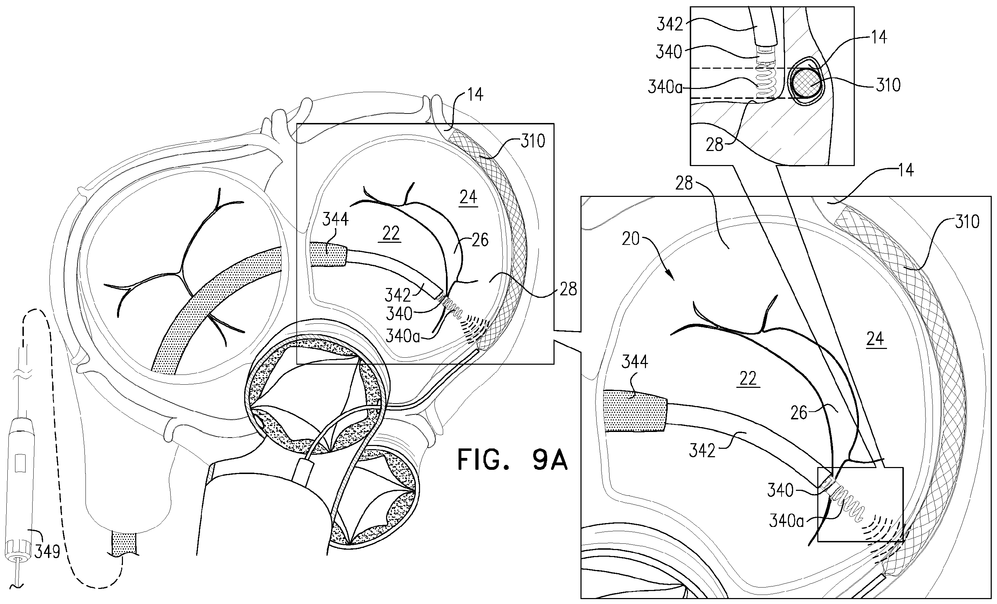

Figs. 9A to 9C are schematic sectional view illustrations of steps of a method of preventing damage to the coronary artery during treatment of a mitral valve of the heart of the subject, in accordance with some applications of the teachings herein. The method can be performed on a living animal or on a simulation, such as on a cadaver, cadaver heart, simulator (e.g., with the body parts, heart, tissue, etc. being simulated), etc.

As seen in Fig. 9A, an elongate tube 310, similar to elongate tube 210 of Figs. 7 to 8K, is advanced into and expanded within left coronary artery 14 surrounding the mitral valve, substantially as described hereinabove with respect to Figs. 7 to 8B. The following description relates to a transluminal annuloplasty procedure, as described hereinabove, but can be used also when implementing other annuloplasty methods and other treatments known in the art, provided that such treatments require insertion of a tissue anchor.

The example of Figs. 9A to 9C is distinct from that of Figs. 8A to 8K in that the elongate tube 310 is not necessarily sufficiently radiopaque to facilitate fluoroscopic guidance, but rather is configured to apply an electrical signal (e.g., a voltage) to the tissue of the heart, for example from under the control of a controller or handle, similar to proximal part 219 of Fig. 8A.

Typically, elongate tube 310 is flexible. In some applications, such as that shown, elongate tube 310 can include, or be formed of, a metal frame or a mesh frame. For treatment of the mitral valve (see reference numeral 20 in Fig. 9A) of the subject, for example having the anatomy described hereinabove with respect to Fig. 8A, elongate tube 310 is transluminally advanced into left coronary artery 14 (e.g., left circumflex artery) which at least partially circumscribes the mitral valve (see for example Fig. 9A).

As seen in Fig. 9A, a first tissue anchor 340, e.g., driven by a driving tool 342 extending distally out of a second catheter 344, is advanced to the left atrium of the heart of the subject, upstream of mitral valve 20, substantially as described hereinabove with respect to Fig. 8C.

In the illustrated example, a distal part of catheter 344 is advanced to the vena cava. Catheter 344 also has an extracorporeal proximal part 349, which can include a handle (e.g., as shown in Fig. 9A). The distal part of catheter 344 is guidable to the anatomical site, such as by being actively steerable (e.g., by being operatively coupled by one or more pullwires to proximal part 349, such as to a steering controller thereof), or by being passively guided and/or steered (e.g., by extending over or through another steerable element, such as an actively steerable catheter).

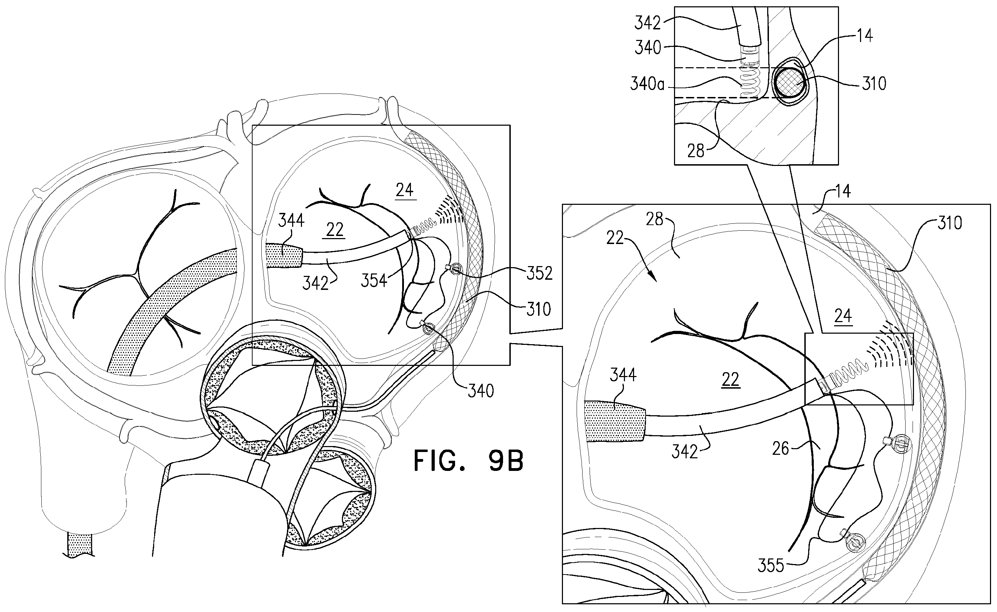

Tissue anchor 340 can have a head that is slidably coupled to a tether (see reference numeral 355 in Fig. 9B), e.g., by the head comprising an eyelet that is threadable onto the tether. Tissue anchor 340 can have a tissue-engaging element, which can be a helical screw-in tissue-engaging element (as described herein) or can be another type of tissue-engaging element, such as a dart or a staple. For some applications, tissue anchor 340 can comprise an anchor described in one or more of the following:
- US Patent Application 17/145,258 to Kasher et al., filed January 8, 2021, which published as US 2021/0145584;
- US Provisional Patent Application 63/147,699 to Shafigh et al., filed February 9, 2021; and
- International Patent Application PCT/IB2022/051099 to Shafigh et al., filed February 8, 2022.

Driving tool 342 is reversibly coupled to the tissue anchor and is configured to detect the electrical signal applied by tube 310. For example, tool 342 can comprise a distal electrode, or can be electrically coupled to the tissue anchor with the tissue anchor serving as an electrode. The detected signal (e.g., its magnitude) is used to determine the proximity of tool 324 and/or the tissue anchor to tube 310, and thereby to coronary artery 14. For example, the signal can be detected when contact is made with tissue of mitral valve 20. For some applications, if an amplitude of the signal is above a predetermined threshold, that is indicative of the tissue anchor being too close to elongate tube 310 and to left coronary artery 14, and a new position is selected, so as to reduce a likelihood of damaging of the left coronary artery when anchoring the tissue anchor 340.

It is to be noted that the technique described with reference to Figs. 9A-C does not necessarily require contact between the tissue anchor and tube 310. That is, the technique does not only alert the end user or physician once the tissue anchor has reached the coronary artery. Rather, the technique can provide general and/or preemptive guidance to the end user physician.

In some applications, driving tool 342 controls tissue anchor 340 such that the tissue-engaging portion 340a engages tissue of the mitral valve at several positions to detect the electrical signal in each position, and the position at which an optimal signal (e.g., a signal having a sufficiently low amplitude) is detected is selected for anchoring of tissue anchor 340.

In some applications, an indication of the electrical signal detected by driving tool 342 is indicated on the proximal part 349 of catheter 344 that is visible to an end user or physician. In some applications, the indication of the electrical signal is displayed on a display screen that is visible to the end user or physician, for example as shown in Fig. 8B.

When a suitable position, which is sufficiently distant from the coronary blood vessel or artery, is detected, driving tool 342 anchors first tissue anchor 340 into tissue of annulus 28 of mitral valve 20. In some applications, driving tool 342 is rotatable, such that rotation of the driving tool screws in a tissue-engaging portion of 340a of first tissue anchor 340 into the tissue of annulus 28.

Turning to Fig. 9B, the first tissue anchor 340 and a second tissue anchor 352 have been anchored into the tissue of annulus 28, and a third tissue anchor 354 is in the process of being anchored, in a similar manner to that described above for the first tissue anchor. Tissue anchors 352 and 354 are similar in structure to tissue anchor 340 described hereinabove, and are slidably coupled to tether 355, which extends distally out of driving tool 342 or out of second catheter 344.

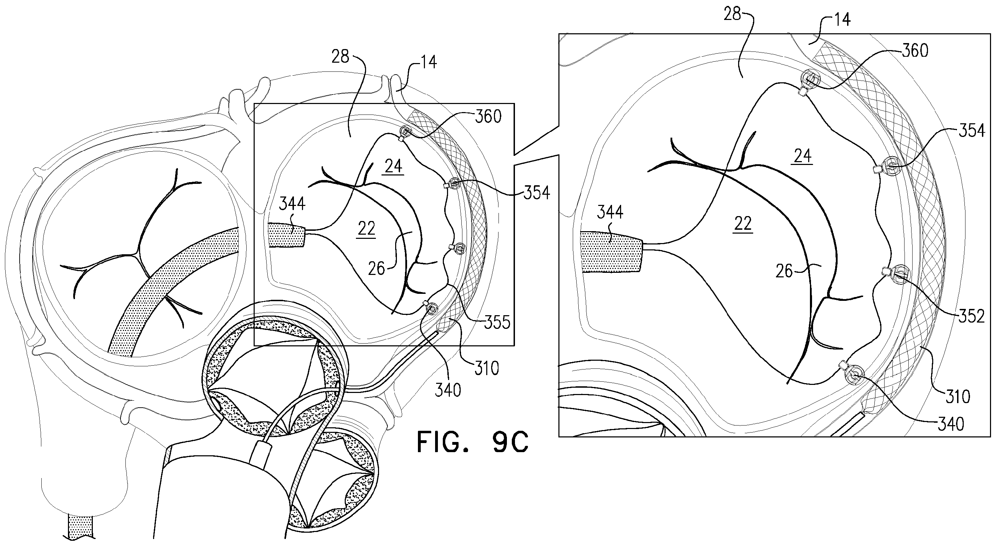

Fig. 9C shows mitral valve 20 following anchoring of tissue anchors 340, 352, and 354 shown previously, and of a fourth, additional tissue anchor 360, similar in structure to tissue anchor 340 and slidably coupled to tether 355. Once the four tissue anchors are installed, the treatment process proceeds in the manner described hereinabove with respect to Figs. 8F to 8K. It is appreciated that while the description of Figs. 9A-9C herein is provided with respect to treatment (e.g., annuloplasty) of the mitral valve, the method and device of the teachings herein can be used, mutatis mutandis, during treatment of the tricuspid valve, by advancing elongate tube 310 into a right coronary artery and carrying out treatment of the tricuspid valve via the vena cava, for example as described hereinabove with respect to Figs. 4 and 5A-5L. In some applications, the elongate tube can be advanced into the right coronary artery via a coronary ostia at the aortic root of the aorta of the subject. In some applications, elongate tube 310 is sufficiently long to extend around at least a majority of the annulus of the tricuspid valve (see for example Figs. 4-5L), and the anchoring of tissue anchors 340, 352, 354, and 360, or any other number of tissue anchors, is into the annulus of the tricuspid valve.

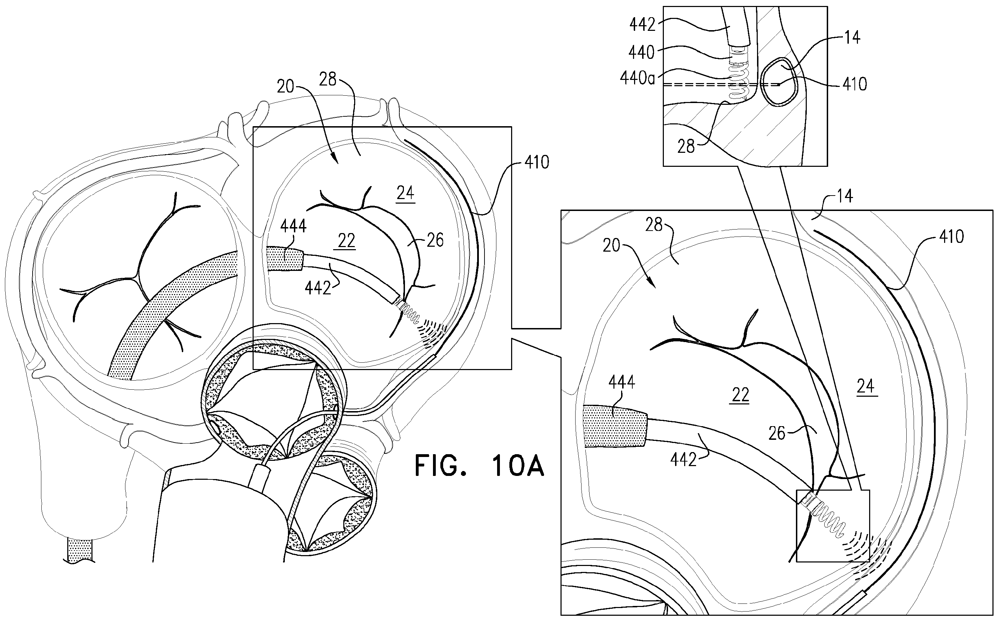

Figs. 10A to 10C are schematic sectional view illustrations of steps of another method of preventing damage to the coronary blood vessel or artery during treatment of a mitral valve of the heart of the subject, in accordance with some applications of the teachings herein. The method can be performed on a living animal or on a simulation, such as on a cadaver, cadaver heart, simulator (e.g., with the body parts, heart, tissue, etc. being simulated), etc.

Figs. 10A-10C demonstrate a process of selecting suitable locations for tissue anchors that is substantially similar to that shown in Figs. 9A-9C. However, in the example of Figs. 10A to 10C, elongate tube 310 is replaced by a wire 410, which typically does not fill the diameter of the coronary blood vessel or artery. In some applications, elongate wire 410 is flexible. It will be appreciated by a people skilled in the art that other elongate, electrical signal-applying structures, may be suitable for implementing the techniques described with reference to Figs. 10A-10C.

Elongate wire 410 is introduced into the left coronary artery substantially as described hereinabove with respect to Figs. 9A to 9C. During anchoring of tissue anchors, elongate wire 410 functions similarly to elongate tube 310 of Figs. 9A to 9C and applies an electrical signal which is detectable during insertion of tissue anchors.

Fig. 10A is substantially similar to Fig. 9A and shows advancement and positioning of a first tissue anchor 440, e.g., driven by a driving tool 442 extending distally out of a second catheter 444, substantially as described hereinabove with respect to Fig. 9A. First tissue anchor 440, driving tool 442, and catheter 444 have structures similar to those of the first tissue anchor, driving tool, and catheter shown and described with respect to Fig. 9A, with like numbers indicating like portions.

As discussed hereinabove with respect to Fig. 9A, driving tool 442 is mechanically and electrically coupled to the tissue anchor, and is configured to detect the applied electrical signal. As such, when a tissue-engaging portion 440a of first tissue anchor 440 engages tissue of mitral valve 20, for example when arriving at a desired anchoring position, the electrical signal is detected by driving tool 442. If the detected electrical signal has a magnitude above a predetermined threshold, that is indicative of the position being too close to elongate wire 410 and to left coronary artery 14, and a new position is selected, so as to avoid damaging of the coronary artery when anchoring the tissue anchor 440.

When a suitable position, which is sufficiently distant from the coronary blood vessel or artery, is detected, driving tool 442 anchors first tissue anchor 440 into tissue of annulus 28 of mitral valve 20. In some applications, driving tool 442 is rotatable, such that rotation of the driving tool screws in a tissue-engaging portion of 440a of first tissue anchor 440 into the tissue of annulus 28.

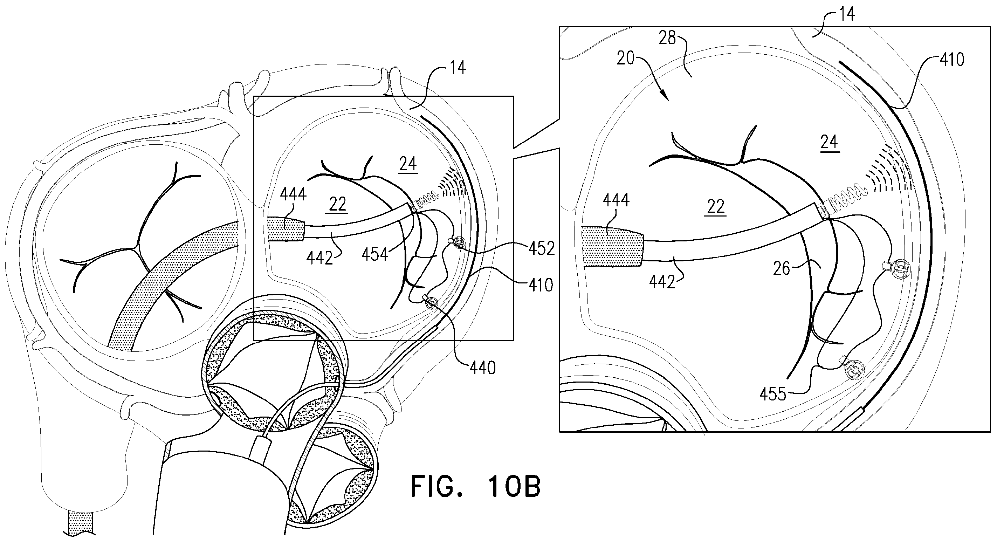

Turning to Fig. 10B, the first tissue anchor 440 and a second tissue anchor 452 have been anchored into the tissue of annulus 28, and a third tissue anchor 454 is in the process of being anchored, in a similar manner to that described above for the first tissue anchor. Tissue anchors 452 and 454 are similar in structure to tissue anchor 440 described hereinabove, and are slidably coupled to tether 455, which extends distally out of driving tool 442 or out of second catheter 444.

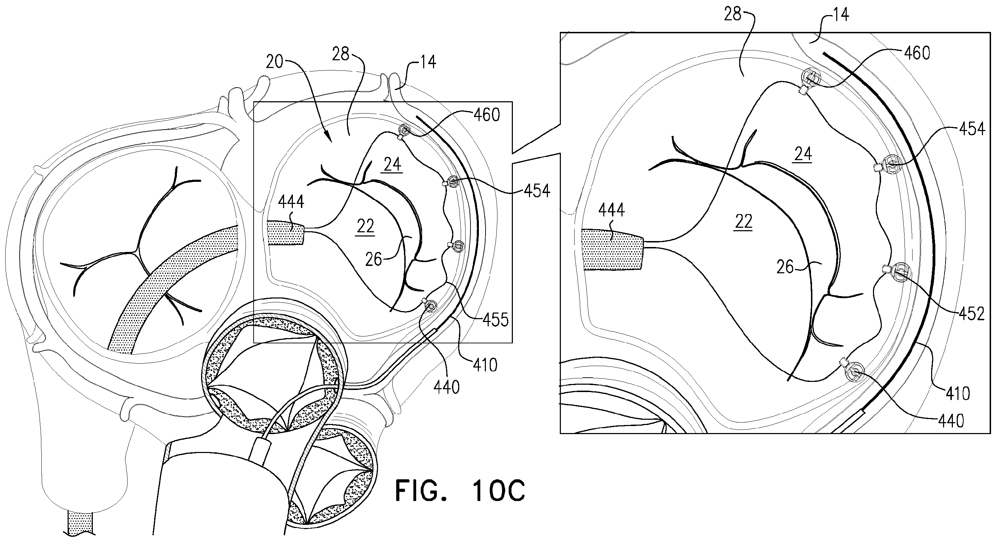

Fig. 10C shows mitral valve 20 following anchoring of tissue anchors 440, 452, and 454 shown previously, and of a fourth, additional tissue anchor 460, similar in structure to tissue anchor 340 and slidably coupled to tether 455. Once the four tissue anchors are installed, the treatment process proceeds in the manner described hereinabove with respect to Figs. 8F to 8K.

It is to be noted that, although the description of Figs. 10A-10C herein is provided with respect to treatment (e.g., annuloplasty) of the mitral valve, the method and device of the teachings herein can be used, mutatis mutandis, during treatment of the tricuspid valve, by advancing elongate wire 410 into a right coronary artery and carrying out treatment of the tricuspid valve via the vena cava, for example as described hereinabove with respect to Figs. 4 and 5A-5L. In some applications, the elongate wire can be advanced into the right coronary artery via a coronary ostia at the aortic root of the aorta of the subject. In some applications, elongate wire 410 is sufficiently long to extend around at least a majority of the annulus of the tricuspid valve (see for example Figs. 4-5L), and the anchoring of tissue anchors 440, 452, 454, and 460, or any other number of tissue anchors, is into the annulus of the tricuspid valve.

The techniques of Figs. 9A-10C are described hereinabove as facilitating distancing of tissue anchors from an adjacent coronary blood vessel or artery, e.g., by anchoring only to tissue sites at which the applied electrical signal is detected at a sufficiently low amplitude. However, it is to be noted that locating tissue anchors within a certain proximity to the atrial wall can be advantageous, e.g., so that the anchors are driven into the annulus of the valve, rather than into the leaflets of the valve. Therefore, for some applications, detection of the applied electrical signal at a sufficiently high magnitude is also desirable, and anchors are only anchored to tissue sites at which the applied electrical signal is detected at an amplitude that is above a given predetermined threshold. Therefore, for some applications, anchors are only anchored to tissue sites at which the applied electrical signal is detected at an amplitude that is between a predetermined lower threshold and a predetermined upper threshold.

Reference is again made to Figs. 2L, 3A, 3B, 5L, 6A, 6B, and 8K. Each of the locks described hereinabove can comprise deformable structure (e.g., a crimp), a spring-loaded element, and/or an actuatable mechanism. For some applications, each of the locks described hereinabove can comprise a lock or a stopper described in one or more of the following:
- US Patent Application 16/534,875 to Brauon et al., filed August 7, 2019, which published as US 2020/0015971;
- International Patent Application PCT/IB2020/060044 to Kasher et al., filed October 27, 2020, which published as WO 2021/084407; and
- US Patent Application 17/145,258 to Kasher et al., filed January 8, 2021, which published as US 2021/0145584.

The present invention is not limited to what has been particularly shown and described hereinabove. Rather, the scope of the present invention is defined by the appended claims. The treatment techniques, methods, operations, steps, etc. described or suggested herein can be performed on a living animal or on a non-living simulation, such as on a cadaver, cadaver heart, simulator (e.g., with the body parts, tissue, etc. being simulated), etc.

Although the operations of some of the disclosed embodiments are described in a particular, sequential order for convenient presentation, it should be understood that this manner of description encompasses rearrangement, unless a particular ordering is required by specific language set forth above. For example, operations or steps described sequentially can in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms can vary depending on the particular implementation and are discernible by one of ordinary skill in the art.

## Claims

1. A system for use with a subject, the system comprising:
a tether (10) configured to be transluminally advanced into a coronary blood vessel (36) of the heart of the subject, the coronary blood vessel (36) at least partially transcribing a heart chamber of the heart, the tether (10) having a first terminus (10a) and a second terminus (10b), the first terminus (10a) configured to be advanced through a wall of the coronary blood vessel (36) into the heart chamber at a first location (14a) and the second terminus (10b) configured to be advanced through the wall of the coronary blood vessel (36) into the heart chamber at a second location (14b);
at least one device (30) configured to transluminally draw the first and second termini (10a, 10b) of the tether (10), from the first and second locations (14a, 14b), respectively, out of the heart chamber, such that a first segment (34) of the tether (10) extends from the first location (14a) through the heart chamber and a second segment (44) of the tether (10) extends from the second location (14b) through the heart chamber;
a support tube (12) defining a lumen, the support tube (12) being configured to be transluminally advanced into the coronary blood vessel (36), the tether (10) extending through the lumen of the support tube (12) and being slidable relative to the support tube (12);
at least one tissue anchor (50, 56; 80), configured to be slid over and along at least one of the first and second termini (10a, 10b), and to anchor the at least one of the first and second termini (10a, 10b) to tissue of the heart chamber, such that a part of the tether (10) extends between the at least one tissue anchor (50, 56; 80) and the coronary blood vessel (36); and
at least one lock (60, 66; 70), configured, following tensioning of the tether (10), to lock tension in the tether (10).

2. The system of claim 1, wherein the at least one tissue anchor (50, 56; 80) comprises a single tissue anchor (80), configured to be slid over and along both the first and second termini (10a, 10b), and to anchor the first and second termini (10a, 10b) to tissue of the heart chamber, such that two parts of the tether (10) extend between the tissue anchor and the coronary blood vessel (36);
preferably wherein the at least one lock (60, 66; 70) comprises a single lock (70) configured to lock the first and second termini (10a, 10b) of the tether (10) adjacent the single tissue anchor (80).

3. The system of claim 1, wherein the at least one tissue anchor (50, 56; 80) comprises:
a first tissue anchor (50) configured to be slid over and along the first terminus (10a) and to anchor the first terminus (10a) to tissue of the heart chamber at a first placement, such that a first part of the tether (10) extends between the first tissue anchor (50) and the coronary blood vessel (36); and
a second tissue anchor (56) configured to be slid over and along the second terminus (10b) and to anchor the second terminus (10b) to tissue of the heart chamber at a second placement, such that a second part of the tether (10) extends between the second tissue anchor (56) and the coronary blood vessel (36); preferably:
wherein the at least one lock (60, 66; 70) comprises a single lock (70) configured to be slid onto the first and second termini (10a, 10b) of the tether (10) and to lock the first and second termini (10a, 10b) of the tether (10) to a locking point adjacent one of the first and second tissue anchors (56);
or alternatively wherein the at least one lock (60, 66; 70) comprises:
a first lock (60) configured to be slid onto the first terminus (10a) of the tether (10) and to lock the first terminus (10a) of the tether (10) to a first locking (60) point adjacent the first tissue anchor (50); and
a second lock (66) configured to be slid onto the second terminus (10b) of the tether (10) and to lock the second terminus (10b) of the tether (10) to a second locking (66) point adjacent the second tissue anchor (56).

4. The system of any one of claims 1 to 3, wherein the heart chamber is a left atrium of the heart, and the coronary blood vessel (36) is a left coronary artery of the subject; preferably:
wherein the left coronary artery is a left circumflex artery of the subject; and/or
wherein the tension in the tether (10) is configured to reshape a mitral valve (20) of the subject.

5. The system of claim 4, wherein the at least one device (30) is configured to draw at least one of the first and second termini (10a, 10b) through at least one of an interspatial septum (38) of the heart and a vena cava of the subject, preferably:
wherein the vena cava is a superior vena cava; or alternatively
wherein the vena cava is an inferior vena cava (36), and wherein the at least one device (30) is configured to draw at least one of the first terminus (10a) and the second terminus (10b) further through a femoral vein of the subject.

6. The system of claim 5, wherein the at least one device (30) is configured to draw the first and second termini (10a, 10b) through one of the superior vena cava and the inferior vena cava (36); or alternatively
wherein the at least one device (30) comprises a first device (30) configured to draw one of the first and second termini (10a, 10b) through the superior vena cava, and a second device (30) configured to draw the other of the first and second termini (10a, 10b) through the inferior vena cava (36).

7. The system of any one of claims 4 to 6, wherein the tether (10) is configured to be advanced into the coronary blood vessel (36) is through a femoral artery and an aorta of the subject.

8. The system of any one of claims 1 to 3, wherein the heart chamber is a right atrium of the heart, and the coronary blood vessel (36) is a right coronary artery of the subject; preferably:
wherein the tension in the tether (10) is configured to reshape a tricuspid valve (120) of the subject.

9. The system of claim 8, wherein the at least one device (30) is configured to draw at least one of the first and second termini (10a, 10b) through a vena cava of the subject; preferably:
wherein the vena cava is a superior vena cava; or alternatively
wherein the vena cava is an inferior vena cava (36), and wherein the at least one device (30) is configured to draw at least one of the first and second termini (10a, 10b) further through a femoral vein of the subject.

10. The system of claim 9, wherein the at least one device (30) is configured to draw the first and second termini (10a, 10b) through one of the superior vena cava and the inferior vena cava (36); or alternatively
wherein the at least one device (30) comprises a first device (30) configured to draw one of the first and second termini (10a, 10b) through the superior vena cava, and a second device (30) configured to draw the other of the first and second termini (10a, 10b) through the inferior vena cava (36).

11. The system of any one of claims 1 to 10, further comprising a first longitudinal catheter (16) configured to be transluminally advanced to the coronary blood vessel (36), and wherein the tether (10) is configured to be advanced to the coronary blood vessel (36) distally out of the first catheter.

12. The system of any one of claims 1 to 11, wherein the at least one device (30) comprises at least one snare (31); preferably:
wherein the at least one snare (31) comprises a single snare (31) configured to draw the first and second termini (10a, 10b); or alternatively
wherein the at least one snare (31) comprises a first snare (31) configured to draw the first terminus (10a) and a second snare (31) configured to draw the second terminus (10b).

13. The system of claim 12, wherein the at least one snare (31) is configured to be transluminally advanced, out of a catheter, to the heart chamber, prior to at least one of the drawing of the first terminus (10a) and the drawing of the second terminus (10b).

14. The system of claim 11 and any one of claims 12 or 13, further comprising at least one second catheter (32), and wherein the at least one device (30) is configured to draw the first and second termini (10a, 10b) through the at least one second catheter (32).

15. The system of any one of claims 11 to 14, wherein the support tube (12) is configured to be advanced into the coronary blood vessel (36) distally out of the first catheter.

## Patentansprüche

1. System zur Verwendung mit einem Subjekt, umfassend:
eine Leine (10), die dazu konfiguriert ist, transluminal in ein Koronargefäß (36) des Herzens des Subjekts vorgeschoben zu werden, wobei das Koronargefäß (36) zumindest teilweise entlang einer Herzkammer des Herzens verläuft, wobei die Leine (10) ein erstes Ende (10a) und ein zweites Ende (10b) aufweist, wobei das erste Ende (10a) dazu konfiguriert ist, durch eine Wand des Koronargefäßes (36) an eine erste Position (14a) in die Herzkammer vorgeschoben zu werden, und das zweite Ende (10b) dazu konfiguriert ist, durch die Wand des Koronargefäßes (36) an eine zweite Position (14b) in die Herzkammer vorgeschoben zu werden;
mindestens eine Vorrichtung (30), die dazu konfiguriert ist, transluminal das erste und das zweite Ende (10a, 10b) der Leine (10) von der ersten beziehungsweise der zweiten Position (14a, 14b) aus der Herzkammer herauszuziehen, so dass ein erstes Segment (34) der Leine (10) sich von der ersten Position (14a) durch die Herzkammer erstreckt und ein zweites Segment (44) der Leine (10) sich von der zweiten Position (14b) durch die Herzkammer erstreckt;
ein Stützrohr (12), das ein Lumen definiert, wobei das Stützrohr (12) dazu konfiguriert ist, transluminal in das Koronargefäß (36) vorgeschoben zu werden, wobei die Leine (10) sich durch das Lumen des Stützrohrs (12) erstreckt und relativ zu dem Stützrohr (12) verschiebbar ist;
mindestens einen Gewebeanker (50, 56; 80), der dazu konfiguriert ist, über und entlang von mindestens einem von dem ersten und dem zweiten Ende (10a, 10b) geschoben zu werden, und zumindest eines von dem ersten und dem zweiten Ende (10a, 10b) an Gewebe der Herzkammer zu verankern, so dass sich ein Teil der Leine (10) zwischen dem mindestens einen Gewebeanker (50, 56; 80) und dem Koronargefäß (36) erstreckt; und
mindestens eine Verriegelung (60, 66; 70), die dazu konfiguriert ist, nach dem Spannen der Leine (10), die Spannung in der Leine (10) zu fixieren.

2. System gemäß Anspruch 1, wobei der mindestens eine Gewebeanker (50, 56; 80) einen einzelnen Gewebeanker (80) umfasst, der dazu konfiguriert ist, über und entlang sowohl des ersten als auch des zweiten Endes (10a, 10b) zu gleiten und das erste und das zweite Ende (10a, 10b) an Gewebe der Herzkammer zu verankern, so dass sich zwei Teile der Leine (10) zwischen dem Gewebeanker und dem Koronargefäß (36) erstrecken;
wobei vorzugsweise die mindestens eine Verriegelung (60, 66; 70) eine einzelne Verriegelung (70) umfasst, die dazu konfiguriert ist, das erste und das zweite Ende (10a, 10b) der Leine (10) benachbart zu dem einzelnen Gewebeanker (80) zu fixieren.

3. System gemäß Anspruch 1, wobei der mindestens eine Gewebeanker (50, 56; 80) umfasst:
einen ersten Gewebeanker (50), der dazu konfiguriert ist, über und entlang des ersten Endes (10a) zu gleiten und das erste Ende (10a) an Gewebe der Herzkammer an einem ersten Ort zu verankern, so dass sich ein erster Teil der Leine (10) zwischen dem ersten Gewebeanker (50) und dem Koronargefäß (36) erstreckt; und
einen zweiten Gewebeanker (56), der dazu konfiguriert ist, über und entlang des zweiten Endes (10b) zu gleiten und das zweite Ende (10b) an Gewebe der Herzkammer an einem zweiten Ort zu verankern, so dass sich ein zweiter Teil der Leine (10) zwischen dem zweiten Gewebeanker (56) und dem Koronargefäß (36) erstreckt;
vorzugsweise:
wobei die mindestens eine Verriegelung (60, 66; 70) eine einzelne Verriegelung (70) umfasst, die dazu konfiguriert ist, auf das erste und das zweite Ende (10a, 10b) der Leine (10) zu gleiten und das erste und das zweite Ende (10a, 10b) der Leine (10) an einem Verriegelungspunkt benachbart zu einem der ersten und zweiten Gewebeanker (56) zu fixieren;
oder alternativ, wobei die mindestens eine Verriegelung (60, 66; 70) umfasst:
eine erste Verriegelung (60), die dazu konfiguriert ist, auf das erste Ende (10a) der Leine (10) zu gleiten und das erste Ende (10a) der Leine (10) an einem ersten Verriegelungspunkt benachbart zum ersten Gewebeanker (50) zu fixieren; und
eine zweite Verriegelung (66), die dazu konfiguriert ist, auf das zweite Ende (10b) der Leine (10) zu gleiten und das zweite Ende (10b) der Leine (10) an einem zweiten Verriegelungspunkt benachbart zum zweiten Gewebeanker (56) zu fixieren.

4. System gemäß einem der Ansprüche 1 bis 3, wobei die Herzkammer ein linker Vorhof des Herzens ist und das Koronargefäß (36) eine linke Koronararterie des Subjekts ist, wobei vorzugsweise:
die linke Koronararterie eine linke Umgehungsarterie des Subjekts ist; und/oder
wobei die Spannung in der Leine (10) so konfiguriert ist, dass eine Mitralklappe (20) des Subjekts umgeformt wird.

5. System gemäß Anspruch 4, wobei die mindestens eine Vorrichtung (30) dazu konfiguriert ist, mindestens eines von dem ersten und dem zweiten Ende (10a, 10b) durch mindestens eines von einem Vorhofseptum (38) des Herzens und einer Hohlvene des Subjekts zu ziehen, vorzugsweise:
wobei die Hohlvene eine obere Hohlvene ist; oder alternativ
wobei die Hohlvene eine untere Hohlvene (36) ist, und wobei die mindestens eine Vorrichtung (30) dazu konfiguriert ist, mindestens eines von dem ersten Ende (10a) und dem zweiten Ende (10b) weiter durch eine Femoralvene des Subjekts zu ziehen.

6. System gemäß Anspruch 5, wobei die mindestens eine Vorrichtung (30) dazu konfiguriert ist, das erste und das zweite Ende (10a, 10b) durch eines von der oberen Hohlvene und der unteren Hohlvene (36) zu ziehen; oder alternativ
wobei die mindestens eine Vorrichtung (30) eine erste Vorrichtung (30) umfasst, die dazu konfiguriert ist, eines von dem ersten und dem zweiten Ende (10a, 10b) durch die obere Hohlvene zu ziehen, und eine zweite Vorrichtung (30), die dazu konfiguriert ist, das andere von dem ersten und dem zweiten Ende (10a, 10b) durch die untere Hohlvene (36) zu ziehen.

7. System gemäß einem der Ansprüche 4 bis 6, wobei die Leine (10) dazu konfiguriert ist, durch eine Femoralarterie und eine Aorta des Subjekts in das Koronargefäß (36) vorgeschoben zu werden.

8. System gemäß einem der Ansprüche 1 bis 3, wobei die Herzkammer ein rechter Vorhof des Herzens ist und das Koronargefäß (36) eine rechte Koronararterie des Subjekts ist; vorzugsweise:
wobei die Spannung in der Leine (10) so konfiguriert ist, dass eine Trikuspidalklappe (120) des Subjekts umgeformt wird.

9. System gemäß Anspruch 8, wobei die mindestens eine Vorrichtung (30) dazu konfiguriert ist, mindestens eines von dem ersten und dem zweiten Ende (10a, 10b) durch eine Hohlvene des Subjekts zu ziehen; vorzugsweise:
wobei die Hohlvene eine obere Hohlvene ist; oder alternativ
wobei die Hohlvene eine untere Hohlvene (36) ist, und wobei die mindestens eine Vorrichtung (30) dazu konfiguriert ist, mindestens eines von dem ersten und dem zweiten Ende (10a, 10b) weiter durch eine Femoralvene des Subjekts zu ziehen.

10. System gemäß Anspruch 9, wobei die mindestens eine Vorrichtung (30) dazu konfiguriert ist, das erste und das zweite Ende (10a, 10b) durch eines von der oberen Hohlvene und der unteren Hohlvene (36) zu ziehen; oder alternativ
wobei die mindestens eine Vorrichtung (30) eine erste Vorrichtung (30) umfasst, die dazu konfiguriert ist, eines von dem ersten und dem zweiten Ende (10a, 10b) durch die obere Hohlvene zu ziehen, und eine zweite Vorrichtung (30), die dazu konfiguriert ist, das andere von dem ersten und dem zweiten Ende (10a, 10b) durch die untere Hohlvene (36) zu ziehen.

11. System gemäß einem der Ansprüche 1 bis 10, ferner einen ersten länglichen Katheter (16) umfassend, der dazu konfiguriert ist, transluminal zu dem Koronargefäß (36) vorgeschoben zu werden, und wobei die Leine (10) dazu konfiguriert ist, distal aus dem ersten Katheter zu dem Koronargefäß (36) vorgeschoben zu werden.

12. System gemäß einem der Ansprüche 1 bis 11, wobei die mindestens eine Vorrichtung (30) mindestens eine Schlinge (31) umfasst; vorzugsweise:
wobei die mindestens eine Schlinge (31) eine einzelne Schlinge (31) umfasst, die dazu konfiguriert ist, das erste und das zweite Ende (10a, 10b) zu ziehen; oder alternativ
wobei die mindestens eine Schlinge (31) eine erste Schlinge (31) umfasst, die dazu konfiguriert ist, das erste Ende (10a) zu ziehen, und eine zweite Schlinge (31), die dazu konfiguriert ist, das zweite Ende (10b) zu ziehen.

13. System gemäß Anspruch 12, wobei die mindestens eine Schlinge (31) dazu konfiguriert ist, transluminal aus einem Katheter zu der Herzkammer vorgeschoben zu werden, vor zumindest dem Ziehen des ersten Endes (10a) und/oder dem Ziehen des zweiten Endes (10b).

14. System gemäß Anspruch 11 und einem der Ansprüche 12 oder 13, ferner mindestens einen zweiten Katheter (32) umfassend, wobei die mindestens eine Vorrichtung (30) dazu konfiguriert ist, das erste und das zweite Ende (10a, 10b) durch den mindestens einen zweiten Katheter (32) zu ziehen.

15. System gemäß einem der Ansprüche 11 bis 14, wobei das Stützrohr (12) dazu konfiguriert ist, distal aus dem ersten Katheter in das Koronargefäß (36) vorgeschoben zu werden.

## Revendications

1. Système destiné à être utilisé avec un sujet, le système comprenant :
une attache (10) conçue pour être avancée par voie transluminale dans un vaisseau sanguin coronaire (36) du coeur du sujet, le vaisseau sanguin coronaire (36) transcrivant au moins partiellement une chambre cardiaque du coeur, l'attache (10) présentant une première extrémité (10a) et une seconde extrémité (10b), la première extrémité (10a) étant conçue pour être avancée à travers une paroi du vaisseau sanguin coronaire (36) dans la chambre cardiaque en un premier emplacement (14a) et la seconde extrémité (10b) étant conçue pour être avancée à travers la paroi du vaisseau sanguin coronaire (36) dans la chambre cardiaque en un second emplacement (14b) ;
au moins un dispositif (30) conçu pour tirer par voie transluminale les première et seconde extrémités (10a, 10b) de l'attache (10), respectivement à partir des premier et second emplacements (14a, 14b), hors de la chambre cardiaque, de telle sorte qu'un premier segment (34) de l'attache (10) s'étend à partir du premier emplacement (14a) à travers la chambre cardiaque et un second segment (44) de l'attache (10) s'étend à partir du second emplacement (14b) à travers la chambre cardiaque ;
un tube support (12) définissant une lumière, le tube support (12) étant conçu pour être avancé de manière transluminale dans le vaisseau sanguin coronaire (36), l'attache (10) s'étendant à travers la lumière du tube support (12) et pouvant coulisser par rapport au tube support (12) ;
au moins un ancrage tissulaire (50, 56 ; 80), conçu pour être glissé sur et le long d'au moins une extrémité parmi les première et seconde extrémités (10a, 10b) et pour ancrer ladite au moins une extrémité parmi les première et seconde extrémités (10a, 10b) au tissu de la chambre cardiaque, de telle sorte qu'une partie de l'attache (10) s'étend entre ledit au moins un ancrage tissulaire (50, 56 ; 80) et le vaisseau sanguin coronaire (36) ; et
au moins un verrou (60, 66 ; 70) conçu, après la mise sous tension de l'attache (10), pour verrouiller la tension dans l'attache (10).

2. Système selon la revendication 1, ledit au moins un ancrage tissulaire (50, 56 ; 80) comprenant un ancrage tissulaire unique (80), conçu pour être glissé sur et le long à la fois des première et seconde extrémités (10a, 10b) et pour ancrer les première et seconde extrémités (10a, 10b) au tissu de la chambre cardiaque, de telle sorte que deux parties de l'attache (10) s'étendent entre l'ancrage tissulaire et le vaisseau sanguin coronaire (36) ;
de préférence ledit au moins un verrou (60, 66 ; 70) comprenant un verrou unique (70) conçu pour verrouiller les première et seconde extrémités (10a, 10b) de l'attache (10) de manière adjacente à l'ancrage tissulaire unique (80).

3. Système selon la revendication 1, ledit au moins un ancrage tissulaire (50, 56 ; 80) comprenant :
un premier ancrage tissulaire (50) conçu pour être glissé sur et le long de la première extrémité (10a) et pour ancrer la première extrémité (10a) au tissu de la chambre cardiaque en une première position, de telle sorte qu'une première partie de l'attache (10) s'étend entre le premier ancrage tissulaire (50) et le vaisseau sanguin coronaire (36) ; et
un second ancrage tissulaire (56) conçu pour être glissé sur et le long de la seconde extrémité (10b) et pour ancrer la seconde extrémité (10b) au tissu de la chambre cardiaque en une seconde position, de telle sorte qu'une seconde partie de l'attache (10) s'étend entre le second ancrage tissulaire (56) et le vaisseau sanguin coronaire (36) ; de préférence :
ledit au moins un verrou (60, 66 ; 70) comprenant un verrou unique (70) conçu pour être glissé sur les première et seconde extrémités (10a, 10b) de l'attache (10) et pour verrouiller les première et seconde extrémités (10a, 10b) de l'attache (10) à un point de verrouillage adjacent à un ancrage parmi les premier et second ancrages tissulaires (56) ;
ou en variante ledit au moins un verrou (60, 66 ; 70) comprenant :
un premier verrou (60) conçu pour être glissé sur la première extrémité (10a) de l'attache (10) et pour verrouiller la première extrémité (10a) de l'attache (10) à un premier point de verrouillage (60) adjacent au premier ancrage tissulaire (50) ; et
un second verrou (66) conçu pour être glissé sur la seconde extrémité (10b) de l'attache (10) et pour verrouiller la seconde extrémité (10b) de l'attache (10) à un second point de verrouillage (66) adjacent au second ancrage tissulaire (56).

4. Système selon l'une quelconque des revendications 1 à 3, la chambre cardiaque étant une oreillette gauche du coeur et le vaisseau sanguin coronaire (36) étant une artère coronaire gauche du sujet ; de préférence :
l'artère coronaire gauche étant une artère circonflexe gauche du sujet ; et/ou
la tension dans l'attache (10) étant conçue pour remodeler une valvule mitrale (20) du sujet.

5. Système selon la revendication 4, ledit au moins un dispositif (30) étant conçu pour tirer au moins une extrémité parmi les première et seconde extrémités (10a, 10b) à travers au moins un élément parmi un septum interauriculaire (38) du cœur et une veine cave du sujet, de préférence :
la veine cave étant une veine cave supérieure ; ou en variante
la veine cave étant une veine cave inférieure (36) et ledit au moins un dispositif (30) étant conçu pour tirer au moins une extrémité parmi la première extrémité (10a) et la seconde extrémité (10b) plus loin à travers une veine fémorale du sujet.

6. Système selon la revendication 5, ledit au moins un dispositif (30) étant conçu pour tirer les première et seconde extrémités (10a, 10b) à travers une veine parmi la veine cave supérieure et de la veine cave inférieure (36) ; ou en variante
ledit au moins un dispositif (30) comprenant un premier dispositif (30) conçu pour tirer une extrémité parmi les première et seconde extrémités (10a, 10b) à travers la veine cave supérieure et un second dispositif (30) conçu pour tirer l'autre extrémité parmi les première et seconde extrémités (10a, 10b) à travers la veine cave inférieure (36).

7. Système selon l'une quelconque des revendications 4 à 6, l'attache (10) étant conçue pour être avancée dans le vaisseau sanguin coronaire (36) est à travers une artère fémorale et l'aorte du sujet.

8. Système selon l'une quelconque des revendications 1 à 3, la chambre cardiaque étant une oreillette droite du coeur et le vaisseau sanguin coronaire (36) étant une artère coronaire droite du sujet ; de préférence :
la tension dans l'attache (10) étant conçue pour remodeler une valvule tricuspide (120) du sujet.

9. Système selon la revendication 8, ledit au moins un dispositif (30) étant conçu pour tirer au moins une extrémité parmi les première et seconde extrémités (10a, 10b) à travers une veine cave du sujet ; de préférence :
la veine cave étant une veine cave supérieure ; ou en variante
la veine cave étant une veine cave inférieure (36) et ledit au moins un dispositif (30) étant conçu pour tirer au moins une extrémité parmi les première et seconde extrémités (10a, 10b) plus loin à travers une veine fémorale du sujet.

10. Système selon la revendication 9, ledit au moins un dispositif (30) étant conçu pour tirer les première et seconde extrémités (10a, 10b) à travers une veine parmi la veine cave supérieure et la veine cave inférieure (36) ; ou en variante
ledit au moins un dispositif (30) comprenant un premier dispositif (30) conçu pour tirer une extrémité parmi les première et seconde extrémités (10a, 10b) à travers la veine cave supérieure et un second dispositif (30) conçu pour tirer l'autre extrémité parmi les première et seconde extrémités (10a, 10b) à travers la veine cave inférieure (36).

11. Système selon l'une quelconque des revendications 1 à 10, comprenant en outre un premier cathéter longitudinal (16) conçu pour être avancé par voie transluminale vers le vaisseau sanguin coronaire (36) et l'attache (10) étant conçue pour être avancée vers le vaisseau sanguin coronaire (36) de manière distale hors du premier cathéter.

12. Système selon l'une quelconque des revendications 1 à 11, ledit au moins un dispositif (30) comprenant au moins une anse (31) ; de preference :
ladite au moins une anse (31) comprenant une anse unique (31) conçue pour tirer les première et seconde extrémités (10a, 10b) ; ou en variante
ladite au moins une anse (31) comprenant une première anse (31) conçue pour tirer la première extrémité (10a) et une seconde anse (31) conçue pour tirer la seconde extrémité (10b).

13. Système selon la revendication 12, ledit au moins une anse (31) étant conçue pour être avancée par voie transluminale, hors d'un cathéter, vers la chambre cardiaque, avant au moins une tirage parmi le tirage de la première extrémité (10a) et le tirage de la seconde extrémité (10b).

14. Système selon la revendication 11 et l'une quelconque des revendications 12 ou 13, comprenant en outre au moins un second cathéter (32) et ledit au moins un dispositif (30) étant conçu pour tirer les première et seconde extrémités (10a, 10b) à travers ledit au moins un second cathéter (32).

15. Système selon l'une quelconque des revendications 11 à 14, le tube support (12) étant conçu pour être avancé dans le vaisseau sanguin coronaire (36) de manière distale hors du premier cathéter.
